# EUROPEAN PATENT APPLICATION

(11) **EP 2 573 185 A2**
(43) Date of publication of application: **27.03.2013**
(21) Application number: 12176017.7
(22) Date of filing: 16.02.2006
(51) Int. Cl.: C12N 15/867, A61K 48/00, C12N 7/02, A61K 39/145

(54) **Lentiviral vectors and their use**

(30) Priority: 16.02.2005 US 653386 P; 10.03.2005 US 660310 P; 18.05.2005 US 682059 P; 05.10.2005 US 723768 P
(62) Divisional of application: 06735210.4
(71) Applicant: Lentigen Corporation, Baltimore, Maryland 21227 (US); Dropulic, Boro, Ellicott City, Maryland 21042 (US); Chang, Yung Nien, Elkridge, Maryland 21075 (US)
(72) Inventor: Dropulic, Boro, Ellicott City, MD 21042 (US); Chang, Yung Nien, Elkridge, MD 21075 (US)
(74) Representative: Pilkington, Stephanie Joan

(57) **Abstract**

The present invention relates to lentiviral vectors for gene therapy, cancer treatment, producing recombinant proteins, such as antibodies and vaccines, and other therapeutic purposes. Novel lentiviral vectors are disclosed, e.g., comprising helper sequences in opposite orientations and/or minimally functional LTR sequences, which can be used to prepare high efficiency transduction vectors. Vectors are also designed to express silencing RNA and antisense polynucleotides.

## Description

This application claims the benefit of U.S. Provisional Application Nos. 60/653,386, filed February 16,2005; 60/660,310, filed March 10, 2005; 60/682,059, filed May 18, 2005; and 60/723,768, filed October 5, 2005, which are hereby incorporated by reference in their entirety.

### BRIEF DESCRIPTION OF THE FIGURES

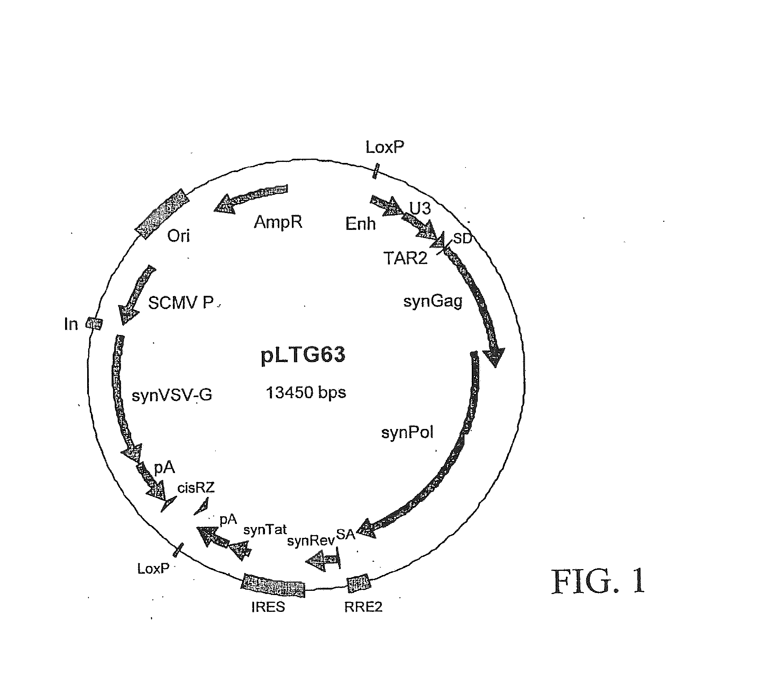

Fig. 1 is a schematic diagram of a helper vector for a two plasmid system containing vsv-g and gag-pol in opposite orientations.

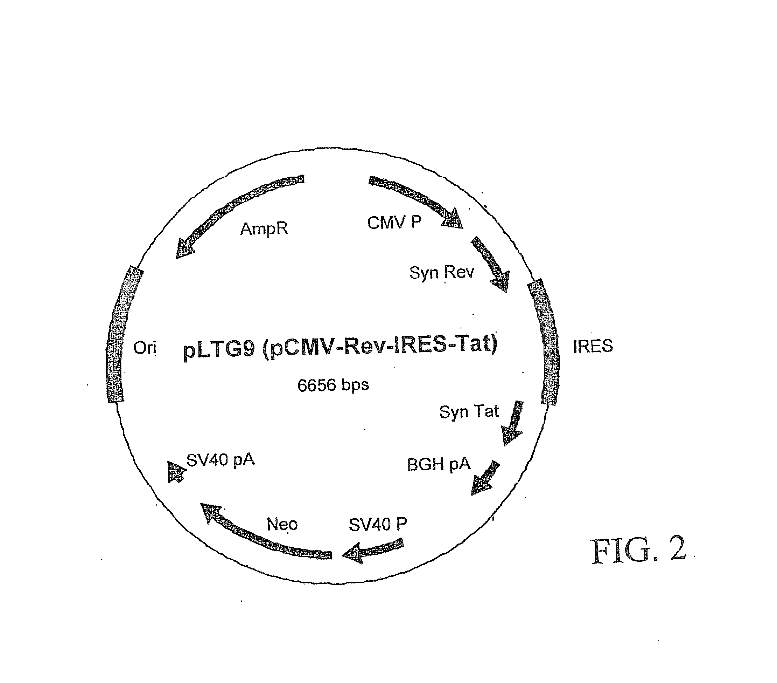

Fig. 2 is a schematic diagram of a transfer vector expressing green fluorescent protein (GFP).

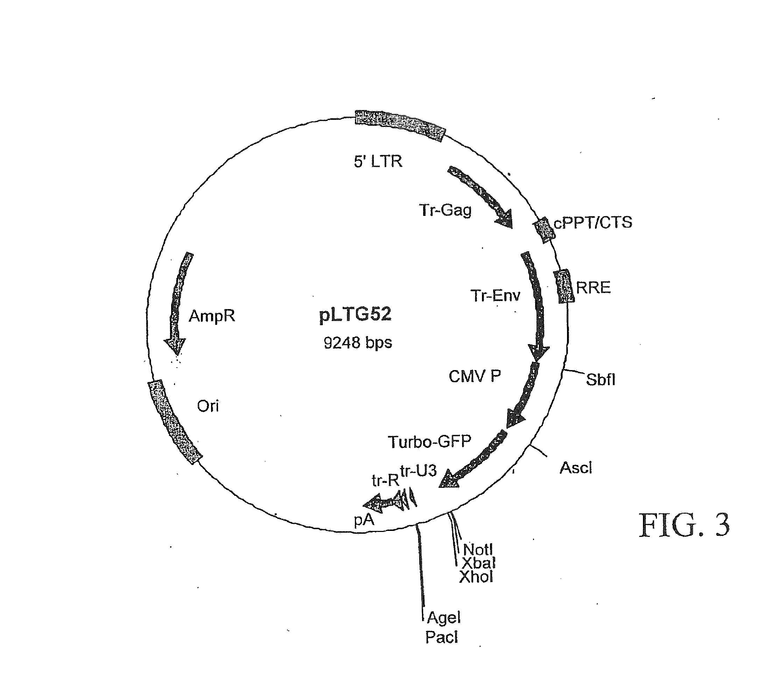

Fig. 3 is a schematic diagram of an expression for Tat and Rev used in a three plasmid system, where the envelope and gag-pol sequences are on another plasmid.

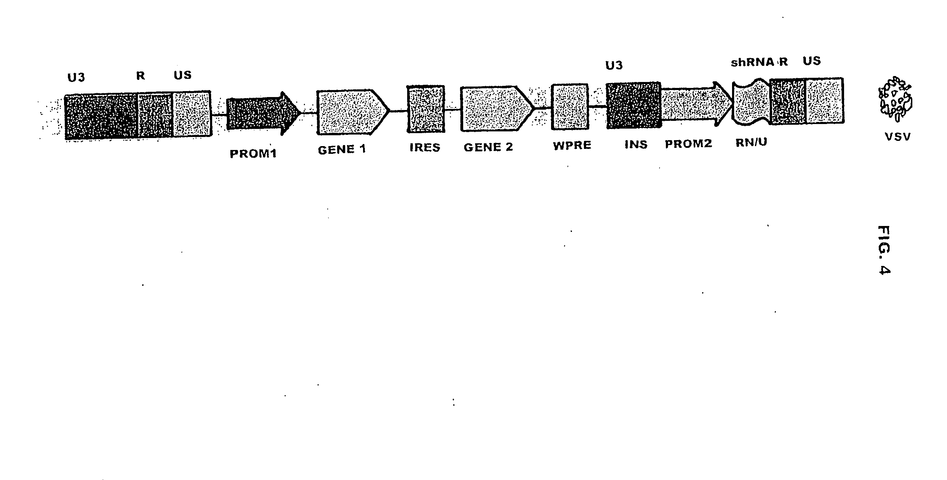

Fig. 4 is an example of a modular transfer vector of the present invention.

### DESCRIPTION OF THE INVENTION

The present invention provides lentiviral vectors, transduction vectors, lentiviral systems, and methods for their use in functional genomics, drug discovery, target validation, protein production (e.g., therapeutic proteins, vaccines, monoclonal antibodies), gene therapy, and therapeutic treatments. Any of the methods disclosed herein can be accomplished with the novel vectors provided by the present invention, or with lentiviral vectors and systems which are known in the art, such as mobilizing vectors (e.g. U.S. Pat. Nos. 5,885,806 or 6,114,141) or non-mobilizing or self-inactivating vectors (e.g. U.S. Pat. Nos. 5,994,136 or 6,428,953).

### Lentiviral transduction vectors

The present invention relates to lentiviral transduction vectors, and constructs for their manufacture, which can be utilized to introduce expressible polynucleotide sequences of interest into host cells. A lentiviral transduction vector is an enveloped virion particle that contains an expressible polynucleotide sequence, and which is capable of penetrating a target host cell, thereby carrying the expressible sequence into the cell. The enveloped particle is preferably pseudotyped with an engineered or native viral envelope protein from another viral species, including non-lentiviruses, which alters the host range and infectivity of the native lentivirus. As described in more detail below, the transduction vectors can be utilized in a wide range of applications, including, e.g., for protein production (including vaccine production), for gene therapy, to deliver therapeutic polypeptides, to deliver siRNA, ribozymes, anti-sense, and other functional polynucleotides, etc. Such transduction vectors have the ability to carry single or dual genes, and to include inhibitory sequences (e.g., RNAi or antisense). In certain embodiments, the transduction vector also carries a nucleic acid which comprises a modified 3' LTR having reduced, but not absent, transcriptional activity.

### Lentiviral helper constructs

The present invention provides lentiviral helper constructs (e.g., a plasmids or isolated nucleic acids). Such constructs contain the elements that are useful for producing a functional lentiviral transduction vector in a compatible host cell, and packaging into it an expressible heterologous sequence. These elements include structural proteins (e.g., the gag precursor), processing proteins (e.g., the pol precursor), such as proteases, envelope protein, and the expression and regulatory signals needed to manufacture the proteins in host cells and assemble functional viral particles. Although the embodiment described below contains the envelope and gag-pol precursor on the same plasmid, they can be placed on separate plasmids, if desired, including separate plasmids for each of the gag, pol, and envelope proteins.

A lentiviral helper plasmid of the present invention can comprise one or more of the following elements in any suitable order or position, e.g., a) lentivirus 5' LTR comprising a functional native promoter operably linked to a polynucleotide sequence coding for lentivirus gag and pol (e.g., a lentivirus gag-pol precursor); and b) heterologous promoter operably linked to an envelope coding sequence. The lentivirus 5'LTR can optionally contain heterologous enhancer sequences located upstream from the native sequence.

Any suitable lentiviral 5' LTR can be utilized in accordance with the present invention, including an LTR obtained from any lentivirus species, sub-species, strain or clade. This includes primate and non-primate lentiviruses. Specific examples of species, etc., include, but are not limited to, e.g., HIV-1 (including subspecies, clades, or strains, such as A, B, C, D, E, F, and G, R5 and R5X4 viruses, etc.), HIV-2 (including subspecies, clades, or strains, such as, R5 and R5X4 viruses, etc.), simian immunodeficiency virus (SIV), simian/human immunodeficiency virus (SHIV), feline immunodeficiency virus (FIV), bovine immunodeficiency virus (BIV), caprine-arthritis-encephalitis virus, Jembrana disease virus, ovine lentivirus, visna virus, and equine infectious anemia virus. Genomic sequence for such viruses are widely available, e.g., HIV-1 (NC_001802), HIV-2 (NC_001722), SIV (NC_001549), SIV-2 (NC_004455), Caprine arthritis-encephalitis virus (NC_001463), Simian-Human immunodeficiency virus (NC_001870), FIV (NC_001482), Jembrana disease virus (NC_001654), ovine (NC_001511), Visna virus (NC_001452), Equine infectious anemia virus (NC_001450), and BIV (NC_001413).

The lentiviral 5' LTR comprises signals utilized in gene expression, including enhancer, promoter, transcription initiation (capping), transcription terminator, and polyadenylation. They are typically described as having U3, R, and U5 regions. The U3 region of the LTR contains enhancer, promoter and transcriptional regulatory signals, including RBEIII,NF-kB, Sp1, AP-1 and/or GABP motifs. The TATA box is located about 25 base pairs from the beginning of the R sequence, depending on the species and strain from which the 5' LTR was obtained. A completely intact 5' LTR can be utilized, or a modified copy can be utilized. Modifications preferably involve the R region, where a TAR sequence is substituted (see below), and/or deletion of all or part of a U5 region. The modified 5' LTR preferably comprises promoter and enhancer activity, e.g., preferably native U3, modified R with a substituted TAR, and native U5.

The 5' LTR can be operably linked to a polynucleotide sequence coding for lentivirus gag and pol. By the term "operably linked," it is meant that the LTR is positioned in such a way that it can drive transcription of the recited coding sequences. The gag and pol coding sequences are organized as the Gag-Pol Precursor in native lentivirus. The gag sequence codes for a 55-kD Gag precursor protein, also called p55. The p55 is cleaved by the virally encoded protease4 (a product of the pol gene) during the process of maturation into four smaller proteins designated MA (matrix [p17]), CA (capsid [p24]), NC (nucleocapsid [p9]), and p6. The pol precursor protein is cleaved away from Gag by a virally encoded protease, and further digested to separate the protease (p10), RT (p50), RNase H (p15), and integrase (p31) activities.

One or more splice donor (SD) sites can be present in the helper plasmid. A splice donor site is typically present between the 3' end of the 5'LTR and the packaging sequence. A downstream splice acceptor (SA) can also be present, e.g., at the 3' end of the pol sequences. The SD site can be present in multiple copies at any effective locations in the vector. The SD can have a native lentiviral sequence, or it can be a mutated copy of it.

Native Gag-Pol sequences can be utilized in the helper vector, or modifications can be made. These modifications (described in more detail below) include, chimeric Gag-Pol, where the Gag and Pol sequences are obtained from different viruses (e.g., different species, subspecies, strains, clades, etc., and/or where the sequences have been modified to improve transcription and/or translation, and/or reduce recombination. In other embodiments of the present invention, the sequences coding for the gag and pol precursors can be separated and placed on different vector constructs, where each sequence has its own expression signals.

The RNA genome of HIV-1 contains an approximately 120 nucleotide Psi-packaging signal that is recognized by the nucleocapsid (NC) domain of the Gag polyprotein during virus assembly. The critical portions of the packaging signal is between the major splice donor (SD) site and the gag initiation codon if the HIV provirus, about distal to the U5 region of the 5' LTR. The packaging signal is functionally absent from the helper plasmid to avoid packaging of functionally active gag-pol precursor into the viral transduction vector. See, e.g., U.S. Pat. No. 5,981,276 (Sodroski et al.) which describes vectors containing gag, but which lack the packaging signal.

Additional promoter and enhancer sequences can be placed upstream of the 5' LTR in order to increase, improve, enhance, etc., transcription of the gag-pol precursor. Examples of useful promoters, include, mammalian promoters (e.g., constitutive, inducible, tissue-specific), CMV, RSV, LTR from other lentiviral species, and other promoters as mentioned above and below.

In addition, the plasmid can further comprise transcription termination signals, such as a polyA signal that is effective to terminate transcription driven by the promoter sequence. Any suitable polyA sequence can be utilized, e.g., sequences from beta globin (mammalian, human, rabbit, etc), thymidine kinase, growth hormone, SV40, and many others.

The helper construct can further comprise an envelope module comprising a heterologous promoter operably linked to an envelope coding sequence. The envelope polypeptide is displayed on the viral surface and is involved in the recognition and infection of host cells by a virus particle. The host range and specificity can be changed by modifying or substituting the envelope polypeptide, e.g., with an envelope expressed by a different (heterologous) viral species or which has otherwise been modified. This is called pseudotyping. See, e.g., Yee et al., Proc. Natl. Acad. Sci. USA 91: 9564-9568, 1994. Vesicular stomatitis virus (VSV) protein G (VSV G) has been used extensively because of its broad species and tissue tropism and its ability to confer physical stability and high infectivity to vector particles. See, e.g., Yee et al, Methods Cell Biol., (1994) 43:99-112.

An envelope polypeptide can be utilized without limitation, including, e.g., HIV gp120 (including native and modified forms), Moloney murine leukemia virus (MoMuLV or MMLV), Harvey murine sarcoma virus (HaMuSV or HSV), murine mammary tumor virus (MuMTV or MMTV), gibbon ape leukemia virus (GaLV or GALV), Rous sarcoma virus (RSV), hepatitis viruses, influenza viruses (VSV-G), Moloka, Rabies, filovirus (e.g., Ebola and Marburg, such as GP1/GP2 envelope, including NP_066246 and Q05320), amphotropic, alphavirus, etc. Other examples, include, e.g., envelope proteins from Togaviridae, Rhabdoviridae, Retroviridae, Poxviridae, Paramyxoviridae, and other enveloped virus families. Other example envelopes are from viruses listed in the following database located on the worldwide web at ncbi.nlm.nih.gov/genomes/VIRUSES/viruses.html.

Furthermore, a viral envelope protein can be modified or engineered to contain polypeptide sequences that allow the transduction vector to target and infect host cells outside its normal range or more specifically limit transduction to a cell or tissue type. For example, the envelope protein can be joined in-frame with targeting sequences, such as receptor ligands, antibodies (using an antigen-binding portion of an antibody or a recombinant antibody-type molecule, such as a single chain antibody), and polypeptide moieties or modifications thereof (e.g., where a glycosylation site is present in the targeting sequence) that, when displayed on the transduction vector coat, facilitate directed delivery of the virion particle to a target cell of interest. Furthermore, envelope proteins can further comprise sequences that modulate cell function. Modulating cell function with a transducing vector may increase or decrease transduction efficiency for certain cell types in a mixed population of cells. For example, stem cells could be transduced more specifically with envelope sequences containing ligands or binding partners that bind specifically to stem cells, rather than other cell types that are found in the blood or bone marrow. Such ligands are known in the art. Non-limiting examples are stem cell factor (SCF) and Flt-3 ligand. Other examples, include, e.g., antibodies (e.g., single-chain antibodies that are specific for a cell-type), and essentially any antigen (including receptors) that is specific for such tissues as lung, liver, pancreas, heart, endothelial, smooth, breast, prostate, epithelial, vascular cancer, etc.

Any heterologous promoter can be utilized to drive expression of the viral envelope coding sequence when operably linked to it. Examples include, e.g., CMV, E1F alpha, E1F alpha-HTLV-1 hybrid promoter, ferritin promoters, inducible promoters, constitutive promoters, and other promoters mentioned herein, etc.

In a preferred embodiment of the present invention, the gag and pol sequences are placed in opposite transcriptional orientations from the envelope sequences. By the latter, it is meant that the direction of transcription is opposite or reversed. This can be achieved by placing the corresponding promoters in opposite directions (i.e., facing each other) or using bi-directional promoters (e.g., Trinklein et al., Genome Research 14:62-66, 2004). This arrangement can be utilized for safety purposes, e.g., to reduce the risk of recombination and/or the production of functional recombinant HIV genomes. Safety is increased with such vectors as there is no possibility that transcriptional read-through would result in a RNA that contains both functional gag-pol and envelope sequences. Transcriptional interference can be prevented by utilizing strong polyadenylation sequences that terminate transcription. Examples of strong transcription termination sequences are known in the art, including, e.g., rabbit beta-globin polyadenylation signal (Lanoix and Acheson, EMBO J.1988 Aug;7(8):2515-22), See, also Plant et al., Molecular and Cellular Biology, April 2005, p. 3276-3285, Vol. 25, No. 8. In addition other elements can be inserted between the gag-pol and envelope coding sequences to facilitate transcriptional termination, such as a cis-acting ribozyme, or an RNAi sequence which are targeted to any putative read-through sequence. Similarly, instability sequences, termination sequences, and pause sites can be placed between the coding sequences.

The helper plasmid can further comprise a TAR element that is obtained from a different lentiviral species, group, sub-species, sub-group, strain, or clade than the 5' LTR and/or the gag and pol sequences that are present in it, i.e., it is heterologous to other lentiviral elements present in the plasmid construct. The TAR is preferably present in the 5' LTR in its normal location, e.g., between the U3 and U5 elements of the LTR, e.g., where the native R is replaced by R' of a heterologous lentiviral species [CONFIRM yes]. Examples of various lentiviral species are listed above from which heterologous TAR elements can be derived.

The TAR element is a trans-activating response region or response element that is located in the 5'LTR (e.g., R) of the viral DNA and at the 5' terminus of the corresponding RNA. When present in the lentiviral RNA, the transcriptional transactivator, Tat, binds to it, activating transcription from the HIVLTR many-fold. Tat is an RNA binding protein that binds to a short-stem loop structure formed by the TAR element.

When a heterologous TAR element is utilized, the 5' LTR can be modified routinely by substituting its native TAR for a TAR sequence from another species. Examples of TAR regions are widely known. See, e.g., De Areliano et al., AIDS Res. Human Retro., 21:949-954, 2005. Such a modified lentiviral 5' LTR can comprise intact U3 and U5 regions, such that the LTR is completely functional. The TAR region or the entire R can be substituted [CONFIRM].

As indicated above, the Tat polypeptide binds to the TAR sequence. The coding sequence for Tat can be present in the helper plasmid, or it can be on another element in the packaging system. For example, it can be integrated into the genome of the cell line utilized to produce the viral transduction vector or present on another plasmid or vector construct introduced into the cell line. Any Tat polypeptide can be utilized as long as it is capable of binding to TAR and activating transcription of the RNA. This includes native Tat sequences which are obtained from the same or different species as the cognate TAR element, as well as engineered and modified Tat sequences.

The helper plasmid can further comprise an RRE element, including an RRE element which is obtained from a different lentiviral species than the 5' LTR or gag and pol sequences. The RRE element is the binding site for the rev polypeptide which is a 13-kD sequence-specific RNA binding protein. Constructs which contain the RRE sequence depend on the rev polypeptide for efficient expression. Rev binds to a 240-base region of complex RNA secondary structure of the rev response element ("RRE") that is located within the second intron of HIV, distal to the pol and gag coding sequences. The binding of rev to RRE facilitates the export of unspliced and incompletely spliced viral RNAs from the nucleus to the cytoplasm, thereby regulating the expression of HIV proteins. The RRE element can be in any suitable position on the construct, preferably following the Gag-Pol precursor in its approximate native position. Similarly for the Tat polypeptide, any suitable rev polypeptide can be utilized as long as it retains the ability to bind to RRE. The coding sequence for rev can be present in the helper plasmid, transfer plasmid, on a separate plasmid, or integrating into the host cell line utilized for transduction vector manufacture. Similarly, coding sequences for tat can be present in the helper plasmid, transfer plasmid, on a separate plasmid, or integrating into the host cell line utilized for transduction vector manufacture.

Any of the sequences which are present in the constructs of the present invention can be modified from their native form, e.g., to improve transcription, to improve translation, to reduce or alter secondary RNA structure, and/or to decrease recombination. Modifications include, e.g., nucleotide addition, deletion, substitution, and replacements. For example, coding sequences for gag, pol, rev, and tat can be modified by replacing naturally-occurring codons with non-naturally-occurring codons, e.g., to improve translation in a host cell by substituting them with codons which are translated more effectively in the host cell. The host cell can be referred to as a compatible cell, e.g., to indicate the sequence modification has its effect when the sequence is expressed in a particular host cell type. In addition, sequences can be modified to remove regulatory elements, such as the packaging sequence. Sequences can also be altered to eliminate recombination sites. Examples of hot spots for recombination are, e.g., disclosed in Zhuang et al., J. Virol., 76:11273-11282, 2002.

Further embodiments include the development of helper systems for the production of Lentiviral vectors and packaging cell lines that can then be developed into producer cell lines for any given vector construct. One such embodiment is the use of cellular proteins to increase Lentiviral vector production. Sam68 belongs to a family of proteins that contain KH domains. Some KH proteins are translational regulators, while others are thought to mediate alternative splicing. Sam68 binds to the Rev response element (RRE) of HIV-1 in vitro and in vivo, and can functionally replace and/or synergize with HIV-1 Rev in RRE-mediated gene expression and virus replication (Modem et al Nucleic Acids Research, 2005, Vol. 33, 873-879). Furthermore, Sam68 was also shown to enhance the activities of the Rev-like proteins of other complex retroviruses. Recently, it has been demonstrated that Sam68 enhances the 3 prime end processing of unspliced HIV-1 RNAs to be exported to the cytoplasm. KH proteins other than Sam68 (i.e. SLM-1, SLM-2, QKI-5, QKI-6 and QKI-7) also enhance Rev/RRE-mediated gene expression. However, among the KH proteins tested, only Sam68 was able to activate constitutive transport element (CTE) -mediated gag gene expression in human cells. When overexpressed in the presence of Rev, Sam68 synergizes with Rev to substantially increase export of RRE containing RNAs from the nucleus. Overexpression of Sam68 in the absence of Rev also facilitates the nuclear export of RRE-containing mRNAs. Therefore to increase the production of HIV-based Lentiviral vectors from producer cells, Sam 68 can be expressed from the helper construct to facilitate Lentiviral vector RNA export into the cytoplasm and increase the production of Lentiviral vector particles. Sam68 could be expressed from helper constructs that are rev dependent or rev independent. The invention is not limited to Sam68 and could target other proteins associated with HIV RNA such as SF2/ASF, hRIP, hRNP A1, p54nrb/PSF and RRE BP49. Conversely, an RNAi targeted to Sam68 or these other proteins could be inserted into a Lentiviral vector to inhibit the export of wild-type HIV RNAs as a form of gene therapy against HIV infection. See, below for more detail on HIV therapies.

### Lentiviral transfer vector

The present invention also provides lentiviral transfer vectors. A transfer vector is a construct which contains the polynucleotide sequences which are packaged into the transducing lentiviral vector. The transfer vectors, when canprising 5' LTR and 3' LTR, can be used for the production of transduction vectors that are capable of integrating into the host genome. Such integration can be prevented, e.g., by mutating the integrase molecule that is present on the helper plasmid in the pol sequence. However, integrating vectors are preferable for long term gene delivery.

A lentiviral transfer plasmid vector of the present invention can comprise one or more of the following components: a) lentivirus 5' LTR polynucleotide sequence; b) packaging sequence (psi) distal to said 5' LTR; and c) modified lentivirus 3'LTR that comprises TATA box sequence, but is lacking 3' U3 sequences 5' to the said TATA box sequences. At least one expressible heterologous polynucleotide sequence can be inserted into the transfer vector, e.g., between the packaging sequence and the U5 region of the 3' LTR.

Any suitable lentiviral 5' LTR sequence can be placed in the transfer vector. Such sequence can be intact and fully native, or it can be modified as described above, e.g., by replacing the TAR sequence with a heterologous TAR sequence (R), or by replacing nucleotides in it with non-naturally-occurring nucleotides to minimize recombination events. The 5' LTR as described earlier has U3, R, and U5 regions which are present, but may be modified in such a way that they retain their functional properties.

A packaging sequence (psi) distal to said 5' LTR can also be present in the transfer vector. This sequence (about 110 nucleotides), which is recognized by the NC domain of the Gag, is utilized in cis to facilitate encapsulation of the heterologous sequence of interest into the transducing vector. See, e.g., Lever et al., J. Virol. (1989), 63: 4085-4087; Amarasinghe et al., J. Mol. Bio. (2001), 314(5):961-970. The psi packaging sequence is relatively autonomous of neighboring sequences. Its position in the transfer vector can be determined routinely. See, e.g., Man and Baltimore, J Virol., 54(2): 401-407,1985 which use a reporter gene to optimize positioning of the packaging sequence.

The transfer vector can also include a lentiviral 3' LTR. The 3' LTR has U3, R, and U5 regions which are flanked by PPT and PBS sequences, respectively. The 3' LTR can be intact and native, but preferably it is modified. Preferably modifications include those produce an LTR which retains a minimal amount of functional activity, e.g., transcriptional (promoter-enhancer) functional activity. Such transcriptional activity can be determined routinely, e.g., using a reporter gene. Examples of modifications that produce LTRs with reduced (as compared to the native 3' LTR) and minimal functional activity include, e.g., deletions which are 5' (upstream) to the TATA box in the U3 region. Such deletions can include, e.g., deletions or modifications of one or more of the following transcriptional regulatory sites, such as RBEIII, NF-kB, and/or Sp1, as well as the PPT site. An example of a 3' LTR with minimal transcriptional activity includes a modified lentivirus 3'LTR that comprises TATA box sequence, but is lacking 3' U3 sequences 5' to the said TATA box sequences or in which the 5' sequences are modified (deletion, substitution, addition) such they are not functionally active. For instance, NF-kB and Sp1 sites can be mutagenized to the point where they are inactive, and/or unable to bind to regulatory proteins. Deletions of the 5' upstream region, include, from about 5, 10, 15, 20, 25, 30, 40, 50, etc., nucleotides from the T nucleotide of the TATA box. The amount of transcriptional activity that remains (when compared to the native LTR) can be, for example, from about 0.1-1%, 0.1-2%, 0.1-5%, 0.1-10%, 0.1-20%, 0.1-25%, 0.5-5%, 0.5-10%, 0.5-20%, 0.5-25%; about 0.1%; about 0.5%; about 1%; about 2%; about 5%; about 7%, about 10%, etc.

The 5' end of the U3 region is necessary for integration (terminal dinucleotide+att sequence). Thus, the terminal dinucleotide and the att sequence may represent the 5' boundary of the U3 sequences which can be deleted. In addition, the transfer vector can comprise RRE sequence which can be located either upstream or downstream of a central poly-purine tract sequence. The RRE or central poly-purine tract sequence can be derived from the native or non-native (heterologous) Lentiviral vector sequences.

The 5' regions (e.g., U3) of the 3' LTR can be functionally disrupted by the insertion of heterologous sequences, including expressible coding sequences, such as expressible shRNA, ribozymes, anti-sense, microRNA's and aptamer sequences. These sequences can be expressed from pol II and pol III (e.g., Human U6, Mouse U6, and Human H1, 7SK) promoters and can be located in the vector genome either in the 3'LTR or upstream from the LTR and downstream from the 5'LTR. For promoters, see, e.g., Werner, T. (1999). Models for prediction and recognition of eukaryotic promoters. Mammalian Genome 10, 168-175.

A modified 3' LTR, however, can retain sequences outside the engineered U3 region, e.g., PPT, R, and U5. As for the 5' LTR, the TAR element in the R region can be replaced with a heterologous TAR sequence from a different lentiviral species or subspecies.

Since viral transcription begins at the 3' end of the U3 region of the 5' LTR, these sequences are not part of the viral mRNA, and a copy thereof from the 3' LTR acts as template for the generation of both LTR's in the integrated provirus. If the 3' LTR copy of the U3 region is altered in a vector construct, the vector RNA still is produced from the intact 5' LTR in the producer cells, but cannot be regenerated in target cells. Transduction of such a vector results in the inactivation of both LTR's in the progeny virus. Thus, the retrovirus is self-inactivating (SIN) and such vectors are known as SIN transfer vectors. See, e.g., Mitta et al., Nucl. Acid Res., 30(21):e113, 2002; Zufferey et al., J. Virol., 72:9873-9880,1998; U.S. Pat. No. 6,428,953 (Naldini et al.)

An expressible heterologous polynucleotide sequence can be inserted into the transfer vector, e.g., between the packaging sequence and the 3' LTR. The expressible sequence is the sequence which is encapsulated into the viral transducing vector, and which is essentially its payload. Any heterologous sequence of interest can be inserted into the transfer vector without limitation, including, sequences coding for therapeutic proteins, enzymes, and antibodies, etc.; siRNA; anti-sense; microRNAs, aptamers; ribozymes, any gene inhibitory or silencing sequence; and any sequence which is to be delivered to a host cell via a lentiviral transducing vector.

The term "expressible" indicates that the polynucleotide sequence is capable of being transcribed and translated in the cell. Sequences that confer expressibility include, e.g., enhancers, promoters, polymerase binding sites, ribosome attachment sites, splice donor and acceptor sites, polyadenylation signals, transcription initiation and termination sequences, etc.

Any of the promoters mentioned above can be utilized to drive expression of the heterologous sequence when operably linked to it. When a vector of the present invention encodes a cytotoxic or cytostatic polypeptide (i.e., a gene that expresses a product deleterious to a host cell), an inducible promoter system is preferably operably linked to its coding sequence so that expression of it can be regulated to minimize host toxicity when gene expression is not required. For example, the tetracycline-regulatable gene expression system (Gossen and Bujard, Proc. Natl. Acad. Sci., 89:5547-5551, 1992) can be employed to provide for inducible expression of a gene when tetracycline is withdrawn from the transferred cell.

Other systems that can be used to inducibly control gene expression are systems that utilize promoter containing response elements. In such a system, the promoter is inactive when bound by a promoter-containing element. An inducer ligand turns the promoter on, e.g., in a quantitative manner, where high concentrations of the inducer are associated with higher transcriptional activity. For example, the RheoSwitch® gene regulation system has three major components: a proprietary RheoCept® protein receptor that binds to the promoter region of the target gene, the target gene to be regulated, and a proprietary small organic molecule ligand inducer. The promoter contains a unique response element to which the receptor binds, and target gene expression is only turned on when the inducer binds to the receptor and activates transcription. See, e.g., Kumar et al., J. Biol. Chem., Vol. 279, Issue 26, 27211-27218, June 25, 2004, "Highly Flexible Ligand Binding Pocket of Ecdysone Receptor: A single amino acid change leads to discrimination between two groups of nonsteroidal ecdysone agonists"). Inducible systems can also be used to increase the safety of vectors by integrating a gene that can kill cells transduced with vector. In this application, an inducible promoter expresses a second gene which, regulates the expression of a second inducible promoter that would then express the "suicide" or safety gene that upon activation, results in the killing of transduced cells. The advantage of a dual regulatory "switch" is that the suicide or safety gene is not expressed until it is first induced, and therefore, if immunogenic, would not be expressed until at least one pro-drug was added to stimulate expression of one of the inducible genes. The other advantage of a dual regulatory switch is that the background expression in the absence of pro-drug will be much lower than if a single switch is employed. At least a second pro-drug would be required to actually kill the cells upon expression of the suicide or safety gene. A non-limiting example is the expression of a transcriptional regulatory protein from the first inducible promoter that then binds and potentiates the second inducible system, which in turn expresses any gene of interest, which preferably is a suicide or safety gene. This non-limiting example is not meant to limit the use of a single inducible promoter system for expression of suicide or safety gems, which are themselves activated by the addition of a pro-drug. Also the example set above are not meant to limit to the use of safety or suicide genes, but any gene or sequence of interest can be expressed from such a dual inducible expression system.

To increase the flexibility of the transfer vector and to create a modular vector system, multiple cloning sites (MCS) can further be incorporated into the vector that facilitate the insertion of a heterologous sequences of interest. This MCS facilitates the introduction of any promoter, a single gene, two genes and optionally a gene inhibitory sequence, such as an antisense, ribozyme, shRNA, RNAi, microRNA, aptamer, transdominant mutant protein or the like. A preferable embodiment is the expression of a gene of interest that has been modified so that its nucleotide sequence is codon degenerated with respect to the endogenous gene in a cell, and additionally, the same vector expresses a gene inhibitory or silencing sequences capable of inhibiting or silencing the native gene of interest. This approach has enormous utility in the understanding the function of various protein domains by expressing the protein of interest that has been modified in these domains, and at the same time expressing a gene inhibitory or silencing sequence that represses or silences expression of the native non-modified gene of interest. This application can also be used in gene therapeutic approaches for the treatment of disease. For example, a Lentiviral vector expressing an RNAi targeted to beta-hemoglobin can repress or silence sickle-hemoglobin in patients with sickle cell anemia. The same Lentiviral vector can also express a normal hemoglobin molecule that has been codon-degenerated at the site targeted by the RNAi. In this way erythroid cells expressing sickle globin can represses sickle globin expression, while expressing native hemoglobin and correct the genetic abnormality. The Lentiviral vector would be delivered into a stem cell population that would give rise to erythroid cells expressing hemoglobin that would eventually become red cells. This approach can be used to treat a wide variety of diseases, including cancer, genetic disease and infectious diseases.

The transfer vector can further comprises other additional elements, e.g., arranged in the following order (with the already described elements): 5' LTR, PBS, packaging sequence, splice donor (SD), origin of replication, optionally a central polypurine tract (PPT), RRE, MCS, splice acceptor (SA), and a modified minimally functional 3' LTR. The expressible heterologous polynucleotide sequence can be inserted in between the splice donor site and the U5 region of the 3' LTR. The transfer vector can also contain one or more SD (naturally-occurring or modified) sites, as described above for the helper vector.

The origin of replication can be used to increase the copy number of the construct when present in a host cell. SV40 ori is commonly used for this purpose, e.g., in cells producing SV40 large T antigen, such as HEK293-T cells.

Other elements which can be provided in the transfer vector and which are 3' to the MCS, include, e.g., a synthetic intron or other sequences utilized to stability mRNA, internal ribosome entry sites (IRES) to facilitate translation of two open reading frames from a single mRNA, selectable markers, and transcription termination signals (e.g., polyadenylation site).

Other elements can be used to facilitate the expression of two open reading frames. One example is the 2A/2B peptide sequence which facilitates cleavage of a polypeptide at a predetermined site (Szymczak et al Nature Biotechnology 22: 589594, 2004). In this way, two polypeptide sequences that are separated by the self-cleaving 2A sequence can be produced from a lentiviral vector from a single open reading frame. Another example is to use Internal Ribosome Initiation Sequences or IRES elements such as those from Picornavirus or Foot and Mouth Disease virus are two non-limiting examples. See, also Donnelly et al., J. Gen. Virol., 82:1013-1025, 2001.

The present invention also provides a transfer vector construct, comprising:, e.g., a) lentivirus 5' LTR comprising a functional native promoter operably linked to a polynucleotide sequence coding for a native lentivirus gag and pol (or a fragment thereof), and a heterologous polyA signal which is effective to terminate transcription driven by said native promoter, wherein a translation termination signal is present downstream of the start of the gag-pol sequence, and b) heterologous promoter operably linked to a heterologous polynucleotide sequence located downstream to the gag-pol sequence.

The present invention also provides expression constructs, comprising: a) lentivirus 5' LTR comprising a functional native promoter operably linked to a polynucleotide sequence coding for a native lentivirus gag and pol (and fragments thereof), and a heterologous polyA signal which is effective to terminate transcription driven by said native promoter, wherein a translation termination signal is present downstream of the start of the gag-pol sequence, b) a splice acceptor site located downstream of the gag-pol sequences and c) a heterologous polynucleotide sequence located downstream to the gag-pol sequence that is operably linked to the 5'LTR promoter.

The transfer vector can comprise any of the elements described above for transfer vectors and/or which typically comprise a lentiviral transfer vector. The gag-pol sequence can be substantially complete, with the insertion of a transcription terminator as described above, but also partial fragments of it can be utilized, e.g., fragments which contain the packaging sequence. The termination signal can be placed anywhere in the gag-pol coding sequences, but preferably at a position where only an incomplete copy of gag coding sequence and where no pol coding sequence is produced. The heterologous polynucleotide sequence can be located downstream of the initiation codon of the gag-pol sequence and in a position that is operably linked to the 5'LTR promoter. Such a position can be determined routinely, e.g., using reporter genes to determine what positions facilitate operable linkage. The heterologous sequence can be inserted into a complete gag-pol coding sequence, downstream from the transcription terminator. Alternatively, the gag-pol sequence can be a partial sequence, and the heterologous sequence can follow the partial sequence and the 3' transcription terminator.

An optional format for the vector expression of microRNA's, shRNAs, and other heterologous sequences, is a vector that contains an intact, but non-functional gag-pol sequences by modifying the gag-pol sequence downstream of the 5'LTR. This modification results in a stop codon that is downsteam of the ATG start site of the gag-pol polypeptide, but does not interfere with the cis acting elements for packaging. [should have a claim on this]. The RNAi, microRNA sequence is inserted downstream of the gag-pol sequence. Including additional cis elements will stabilize the vector leading to increased titers and production of functional effector sequences. In another embodiment, such a vector expresses RNAi, microRNAs or shRNAs (antisense etc) that is targeted to multiple sites to increase the probablility that a single effector RNAi effectively inhibits the expression of the target sequence. [should have a claim on this as well]

To inactivate translation or transcription of the pol sequences, polynucleotides can also be inserted between the gag and pol coding sequences, e.g., heterologous sequences heterologous expression cassettes (e.g., promoter, coding sequence, and polyA), siRNA, antisense, translation (e.g., a termination codon) and/or transcription termination sequences. Termination of protein synthesis or translation occurs on ribosomes as a response to a stop codon. Examples of stop codons include, e.g., UAG, UAA, and UGA. See, also, Cassan and Rousset, "UAG readthrough in mammalian cells: Effect of upstream and downstream stop codon contexts reveal different signals," BMC Molecular Biology 2001, 2:3.

### Lentiviral packaging system

The present invention also provides lentiviral packaging systems for producing lentiviral transduction vectors. A packaging system refers to a plurality of constructs which are useful for manufacturing fully-enveloped and functional lentiviral transduction vectors. These include, e.g., a lentiviral helper construct and transfer construct (e.g., in the form of plasmids) as described in detail above (i.e., a two-plasmid, three plasmid or multiple plasmid systems). The helper construct preferably contains both the gag-pol precursor and the envelope protein, but each can also be present on a different construct. In such case, both helper constructs could be included in the system.

In addition, the system can further include constructs for expressing polypeptides that act in trans to enhance production of the transduction vector. These include, preferably plasmids which comprise expressible rev and tat polypeptides for interacting with the RRE and TAR sequences, respectively. Once again, they can be present on the same plasmid, e.g., where each has its own transcription termination signal, or where the coding sequences are separated by an IRES sequence to achieve translation using the same messenger RNA. For example, the system can comprise three plasmids or constructs, including a helper plasmid, transfer plasmid, and a plasmid for expressing the rev and/or tat polypeptides.

Other polypeptides normally present in lentiviruses, such as the accessory proteins nef, vif, vpr, and vpu, are preferably not expressed on any construct present in the transduction system. Optionally, the vpx protein from SIV could be expressed from the vector plasmid, the helper or one of the helper plasmids, or expressed from a plasmid that singly or in combination with another sequence. The vpx protein may facilitate an increase in the transduction efficiency of HIV or other Lentiviral based vectors.

Constructs of the present invention can also comprise origins of replication (e.g., pUC to merit high-copy replication and maintenance in E. coli), selectable markers, and other sequence, e.g., for producing the helper and transfer constructs in bacteria. Additionally, markers can be utilized to assay for the presence of the vector, and thus, to confirm infection and integration. The presence of a marker gene also ensures the selection and growth of only those host cells which express the inserts. Typical selection genes encode proteins that confer resistance to antibiotics and other toxic substances, e.g., histidinol, puromycin, hygromycin, neomycin, methotrexate etc. and cell surface markers.

The helper and transfer vectors of the present invention can exclude the vectors and one or more elements thereof which are described or claimed in, e.g., U.S. Pat. Nos. 5,994,136, 6,165,782, and 6,428,953 (Naldini); U.S. Pat. No. 6,013,516 (Verma); U.S. Pat. Nos. 5,665,577 and 5,981,276 (Sodroski); U.S. Pat. No. 5,817,491 (Yee); U.S. Pat. No. 6,555,107; U.S. Pat. No. 6,627,442; U.S. Pat. No. U.S. 6,051,427 (Finer et al.); U.S. Pat. No. 6,924,123 (Kingsman et al.); U.S. Pat. No. 5,591,264 (Barber et al.).

### Vector construction

Further provided is a mechanism to increase the safety of a Lentiviral vector by including helper sequences into the Lentiviral vector construct. It is known that retroviruses containing direct repeats are unstable and that the level of unstability is directly proportional to the length of the direct repeat sequence. Direct repeat sequences greater than 200 bases are very efficiently excised from a human retrovirus, such as a human lentivirus. By providing a helper sequence from a undesirable helper construct upstream from a possible site of recombination between the vector and helper sequences, the safety of a Lentiviral vector can be improved. For example, it will be undesirable that a VSV-G sequence is incorporated into the Lentiviral vector. A preferred embodiment is to place 500-1000 bases of the 3' or distal region of VSV-G (preferably not including the poly A site) into the vector located upstream from a potential site of recombination (for example, just distal to the Lentiviral vector packaging site). If recombination between the VSV-G sequences from the helper and the vector should occur, then a direct repeat sequence would form, resulting in instability, and its subsequent deletion from the vector during reverse transcription.

Other embodiments are inducible production systems that contain target protein mRNAs that are stabilized with RNA sequences in an inducible manner. For example, the 3' RhoB untranslated region (UTR) can stabilize target RNAs that express either toxic proteins or other proteins of interest in response to serum. Another example is linking the eotaxin 3' untranslated region to the target gene of interest, which normally has a low half-life, but is stabilized with the addition of TNF-alpha and IL-4 to the cells. Alternatively, sequences contained in 16 mer sequence in the 5' coding region of CYP2E 1 and CYP2B 1 mRNA destabilizes target RNAs in the presence of insulin. Upon the removal of insulin the target RNAs are stabilized and the proteins can be expressed (Trong et al. Biochem J. 2004 Dec 23). The preferred invention is to use such destabilization sequences to produce a packaging cell line that can produce toxic proteins like VSV-G in an inducible manner. By linking the destabilization sequences with VSV-G or other protein and either adding or removing a stabilizing factor, inducible expression of the VSV-G or other protein can be achieved. Preferred embodiments are helper constructs that express a toxic proteins containing RNA sequences that destabilize the toxic protein encoding mRNA, yet are stabilized in response to some stabilizing factor. Further preferred embodiments are Lentiviral vectors that encode a protein gene of interest linked to an RNA instability sequence that can be stably expressed upon the addition of some factor that stabilizes the mRNA.

Another embodiment is a Lentiviral vector packaging cell line that expresses an RNAi targeted to the VSV-G protein under an inducible promoter system. During selection of a cell line the anti-VSV-G RNAi is active and is then induced to 'shut-off' to initiate Lentiviral vector production. Such inducible promoters are know in the art and are also described in this application (Gossen, M., and Bujard, H., "Tight Control of Gene Expression in Mammalian Cells by Tetracycline-responsive Promoters," Proc. Natl. Acad. Sci. USA (1992) 89:5547-5551). Other have used the inducible system to induce the expression of VSV-G in packaging cell lines (Yang et al., US patent 5,750,396; Verma US patent 6,218,181). However an alternative method to control the expression of toxic proteins like VSV-G by placing an inhibitor of gene expression that is targeted to the toxic protein under the control of an inducible promoter, such as the tetracycline responsive promoter, but this particular inducible system is not a limitation and other inducible systems could be used. The inhibitor of gene expression can be an antisense, an RNAi (of which there are several variants, some described above), a ribozyme or a transdominant mutant protein that itself is not toxic. A preferred embodiment is the inducible expression of ddRNAi for inhibition of VSV-G expression during maintenance of the cell line which is then "switched-off' during the time of vector production. The same method could be used to induce the expression of a wide variety of proteins during specific phases of cell growth and for applications other than vector production. For example, the expression of the RNAi could be timed with the expression of a cell cycle inhibitor or a second RNAi targeted to a gene that promotes cell cycling or cell division. Other sequences that could be targeted are genes involved in cell death, division, metabolism, protein synthesis and metabolism, cell cycling, nucleic acid synthesis and metabolism and cell differentiation, among other potential target genes. This would be accomplished by operably linking the RNAi that is targeted to the toxic or unwanted protein with a gene using an Internal Ribosomal Entry Sequence (IRES) or a similar sequence that is known in the art. The RNAi could also be linked with a second RNAi simply by separating the two RNAi sequences with a buffer sequence. Buffer sequences are known in the art and they are any sequence which does not interfere with the function of the RNAi sequence.

The above method can be used in the production of safer helper vector systems for production of Lentiviral vectors where the RNAi or an RNA instability sequence is used to prevent toxic or unwanted recombinants of the Lentiviral vector. The RNAi can be targeted to single or multiple regions of potential read-though between open reading frames in the helper construct. The RNA instability sequence (also known as mRNA- and protein-destabilizing elements- e.g., PEST sequences, P1, P2, cUb and Ub, 1, 2 or 4 copies of the nonamer UUAUUUAUU (SEQ ID NO:1) (N1, N2 and N4, respectively), AU-rich elements (ARE) from the *c-fos* and *c-myc* 3'-UTR. Preferred embodiments are double-destabilized constructs which consist of at least one RNA destabilizing element and at least one protein destabilizing element) can be inserted into regions between genes where it would be undesirable to have read-though. For example it would be undesirable to have a VSV-G envelope and Gag or Pol protein on the same mRNA and therefore a RNAi targeted to a single or multiple regions between (or putative areas of recombination) of the VSV-G and the Gag or Pol open reading frames on the helper construct would be a preferred embodiment to the invention. A preferred embodiment is the use of a shRNAi or a ddRNAi targeted to a region on the helper construct that potentially results in a RNA sequence that contains Gag and/or Pol, and VSV-G envelope proteins should read-though occur. The RNA or protein instability or degradation sequences could be used to prevent a read-through transcript or a read through protein sequence by inserting such instability elements or degradation sequences between coding sequences where it would be undesirable for read-though RNA and/or protein sequences to occur. The degradation sequences could be places in all open reading frames and therefore may be repeated at least three times; as the actual reading frame that would be used is not necessarily be known a priori to the read-though or recombination event. Also provided is a method to prevent the envelope and gag-pol open reading frames producing a readthrough polyprotein by ensuring that the gag-pol and vsv-g are in different phases of the triplet codon sequence. Preferably the vsv-g is downstream of the gag-pol and phased -1 to the gag-pol codon triplet sequence.

In another embodiment, the safety of a Lentiviral vector can be increased by inserting an inducible RNAi or antisense sequence that is targeted to any sequence considered to be adverse if it would recombine with the vector. For example, an anti-vsv-g sequence (i.e., an anti-envelope polynucleotide sequence, such as RNAi or anti-sense) could be inserted upstream from the major splice acceptor site so that it is only expressed late during vector production and only in the genomic vector RNA. In this way, it would not significantly affect vector titer. However, if a recombination event should ensue, then the RNAi or antisense sequence would bind to the VSV sequence and destroy the recombinant. Thus, a helper (or transfer vector) can further comprise an anti-sense polynucleotide that is effective to inhibit translation of said envelope coding sequence. The design of antisense are well known in the art, and can comprise the complete antisense sequence inserted into the vector, or a partial sequences thereof which is sufficient to hybridize to the envelope sense RNA and inhibit its translation.

Another embodiment is the presence of the following peptide sequences in Lentiviral vectors or helper expression constructs, KETWETWWTE (SEQ ID NO:2). This peptide sequence is a powerful inhibitor of reverse transcriptase dimerization. The peptide can be used in two formats: for the production of safer Lentiviral vectors from packaging systems, or for HIV/AIDS gene therapy. For the production of safer packaging systems, the peptide is inserted between the gag-pol and envelope (e.g. VSV-G) coding sequences and is expressed only upon readthrough between the two open reading frames. The peptide is then produced to inhibit viability of the vector by inhibiting reverse transcriptase dimerization and packaging into the virion. In the second format it is expressed from HIV based Lentiviral vector for the treatment of HIV/AIDS. Vector containing cells expressing the peptide produce defective particles without dimerized reverse transcriptase upon infection with wt-HIV. This allows for stimulation of the immune response with the epitopes that are present in the body without infectious virus being produced. In a third format, the peptide can be expressed from a Lentiviral vector as a second gene to prevent the vector from any further mobilization after initial transduction. The peptide sequence or multiples of the sequence would only be expressed in the target cell and not during production as the peptide would be dissociated from its promoter sequence in the vector during production, but where the peptide would be produced in the target cell as a result of an intervening direct repeat sequence reassociating the promoter with the peptide sequence to be expressed. The same method could be used to express toxic proteins instead of the peptide that inhibits reverse transcriptase dimerization. Temporally the vector is organized as follows: a 5' LTR derived from a Lentivirus, a packaging sequence, an internal promoter, a sequence not less than 500 bases (preferably but not limiting) containing a splice donor site at its 5' boundary and a strong splice acceptor site, an intervening sequence, the same not less than 500 base sequence without the splice donor site but with a single or multiply point mutated splice acceptor site that is weaker than the strong acceptor site, a codon initiation sequence, the peptide coding sequence (or toxic protein), a codon stop sequence, a poly A, and a 3' LTR derived from a Lentivirus.

Recently the LMO2 gene has been implicated in the development of Leukemias, but appears that this gene is not essential during T cell development (McCormack MP, Forster A, Drynan L, Pannell R, Rabbitts TH Mol Cell Biol. 2003 Dec;23(24):9003-13.) A preferred embodiment is a Lentiviral vector that expresses an antisense, ribozyme, RNAi or an inhibitor LMO2 gene expression to increase the safety of Lentiviral vectors or retroviral vectors during human gene therapy of disease where the CD34 or a hematologic cell type is transduced with a Lentiviral or retroviral vector, where the Lentiviral or retroviral vector integrates into the chromosome of the said cell.

In addition to LMO2, other genes have been shown to be upregulated or downregulated when transduced with HIV vectors (Zhao et al Gene Therapy 12:311-319, 2005). For example, EEF1 alpha is upregulated 10x in human umbilical vein endothelial cells, while Clusterin is upregulated 3x. To prevent any adverse effects due to overexpression of these genes, a Lentiviral vector can be constructed that expresses an RNAi to the overexpressed genes or one could encode and express the genes that are underexpressed. In this way the safety of Lentiviral vectors could be increased.

In addition, specifications are provided for a lentiviral vector where all the codon initiation sites have been deleted using either a point deletion, two base deletions, three base deletions or greater than three base deletion around and including the codon initiation sequence for lentiviral proteins. In this way the vector retains cis acting sequences required for maximum encapsidation, but does not have the ability to produce a wild-type lentivirus. Furthermore, cryptic codon initiation sites are also deleted. In a preferred embodiment, sufficient sequence is deleted surrounding the codon initiation sites to create space for the insertion of the above genes or RNAi to increase the potency of the vector's therapeutic effect or desired non-therapeutic effect- e.g. increased protein production in cell lines.

The advantages of this is that cellular proteins are not immunogenic so that their overexpression will not lead to an immune response against cells containing the vector but as yet not infected with a wild-type lentiviral.

Further is provided the above vectors that express a plurality of genes or RNAi that results in either (1) activation of the cell and increased production of defective vector particles from the cell; (2) stimulation of the immune response; (3) increased production of defective particles; and/or (4) decreased production of infectious lentivirus particles. Such genes or RNAi are described above.

### Transduction vector manufacture

The present invention also provides transduction vectors and methods of producing them. The particular embodiments described above can be used transiently in host cells to produce transduction vectors. Examples of host cells which can be utilized to produce the vectors, include, any mammalian or human cell line or primary cell. Non-limiting examples include, e.g., 293, HT1080, Jurkat, and SupT1cells. Other examples are CHO, 293, Hela, VERO, L929, BHK, NIH 3T3, MRC-5, BAE-1, HEP-G2, NSO, U937, Namalwa, HL60, WEHI 231, YAC 1, U 266B1, SH-SY5Y, CHO, e.g., CHO-K1 (CCL-61),293 (e.g., CRL-1573).

The present invention provides methods for producing a lentivirus transduction vector comprising, e.g., a) transfecting a host cell with a lentivirus helper plasmid and transfer plasmid to produce a producer cell line; and culturing said transformed producer cell under conditions effective to produce a lentiviral transduction vector. Any suitable transfection methods can be used in the vector manufacturing process including electroporation, calcium phosphate transfection, PEI polymer mediated transfection, fecturin or lipid-based transfection methods. The transduction vector is preferably secreted into the cell culture medium where it can be recovered and optionally enriched or purified.

The cell line utilized to manufacture the transduction vector can be modified in any of the ways mentioned below to enhance vector protein production, e.g., by the introduction of RNAi or antisense to knock-out genes that reduce the expression of genes that limit vector production, or by the introduction of sequences that enhance vector production. Sequences that code for cellular or viral enhancers can also be engineered into cell lines (e.g., using additional plasmid vectors), such as herpes virus, hepatitis B virus, which act on HIVLTRs to enhance the level of virus product, or cellular transactivator proteins. Cellular transactivation proteins include, e.g., NF-kB, UV light responsive factors, and T cell activation factors.

The cell lines can be transformed routinely with construct DNA, e.g., using electroporation, calcium phosphate, liposomes, etc., to introduce the DNA into cells. Cells can be co-transformed (i.e., using both helper and transfer vectors), or they can be transformed in separate steps, where each step involves the introduction of a different vector.

Cells are cultured under conditions effective to produce transduction vectors. Such conditions include, e.g., the particular milieu needed to achieve protein production. Such a milieu, includes, e.g., appropriate buffers, oxidizing agents, reducing agents, pH, co-factors, temperature, ion concentrations, suitable age and/or stage of cell (such as, in particular part of the cell cycle, or at a particular stage where particular genes are being expressed) where cells are being used, culture conditions (including cell media, substrates, oxygen, carbon dioxide, glucose and other sugar substrates, serum, growth factors, etc.).

### Transduction efficacy

In addition to the envelope modifications described above, stimulation of cells for increased transduction is not limited to expression of the ligands on the surface of the cells. Transduction efficiency can be further increased in vitro or in vivo by transducing the cells with at least two types of vectors. The first vector is termed a "facilitating vector" where the said vector produces proteins or ligands that stimulate the target cells to be more receptive to incorporate the transducing vector that expresses the therapeutic or other sequence of interest. The facilitating vector can further comprise a safety or suicide gene in addition to the protein, ligand or factor that is used to stimulate the target cells for high efficiency vector mediated transduction. In this way, the facilitating vector can express the proteins, surface ligands, or factors required for high efficiency transduction by the transduction vector, and then be deleted from the target mixture of cells, once the transducing vector has mediated high efficiency transduction of the target population of cells. This method may be used for the transduction of stem cells, where at least one facilitating vector can express a combination of SCF, TPO and Flt-3 ligands, whereby each facilitating vector contains a safety or suicide gene(s) that will eliminate the cells from the population once a pro-drug is added to the population of cells. Safety or suicide genes are know in the art and are described in more detail later in this application. Optionally, the facilitating vector can express the protein, factors or ligands from an inducible promoter that could be used solely or in combination with the safety or suicide gene(s). Layering an inducible system in concert with a safety or suicide gene(s) can be used to increase the sensitivity and specificity (inducible systems can be made to be tissue specific) of protein/factor/ligand/RNAi/antisense etc production, and the expression of the safety or suicide gene(s). Expression of the protein/factor/ligand/RNAi from the facilitating vector can optionally be expressed from a tissue specific promoter, to limit expression of the sequences in the facilitating vector to specific cell types. In a preferred embodiement, the facilitating vector is added to a population of cells with minimum stimulation so that non target cells are preferentially transduced to express the target cell stimulating factors and yet marked with the safety or suicide gene so that they can be deleted at a later date. After a period of time (at least 1 hour and up to several weeks after addition of the facilitating vector, but preferably the next day), the transducing vector is added to the cells for high efficiency mediated transduction.

### Cell lines

The present invention also provides for the development of cell lines that have enhanced properties for growth, reduced dependency upon expensive factors that are present in media, produce higher yields of proteins, and produce higher titers of vector particles. For example it has recently been reported HEK 293 cells have a specific increased expression of cellular receptors and by adding the specific ligands to the medium of the cells, they demonstrated increase proliferation potential (Allison et al., Bioprocess International 3:1, 38-45, 2005). A preferred embodiment is a plurality of Lentiviral vectors expressing an optimized combination of ligand proteins that are of relevance to HEK 293 cells after which the cells are then sorted by high throughput methods to isolate a clone of HEK 293 cells that contains multiple copies of lentiviral vectors. These cells contain a combination of HIV vectors that express different but also multiple copies of the ligand genes that are contained in the HIV vectors. The ligand genes could be codon optimized or mutations added to further increase their expression. A preferred combination is to have multiple copies of the ligand proteins expressed in the final isolated clonal cell that could then have multiple uses. It could be used for protein or antibody (including monoclonal, humanized, single-chain) production. It could also be used for the production of a vector such as a Lentiviral vector, but not limited to a Lentiviral vector. Other vectors such as Adeno and Adeno-associated vectors, murine retroviral vectors, SV40 vectors and other vectors could just as easily be produced from this now optimized cell line. A list of the receptors and their ligands that show increased expression/activity in HEK 293 cells, includes, e.g., AXL receptor (gas6); EGF receptor (EGF), chemokine receptor (fractalline); PDGF receptor, beta (PDGF); IL-15R-alpha; IL-2R-alpha; chemokine receptor 2 (MCP1); IL-2R, gamma; IL-1R-1; CSF-1 receptor; oncostatin receptor; IL-4R; vitamin D3 receptor; neuropilin 1 (VEGF); macrophage stimulating receptor 1 (MSP); NGF-R; PDGFR-alpha receptor; IL-11-R, e.g., alpha; IL-10-R, e.g., beta; FGF-R-4 (aFGF); BMP receptor, e.g., type II (BMP-2); TGF-R, e.g., beta receptor II (TGF-beta); FGF-R-1 (bFGF); chemokine receptor 4 (SFD1a); interferon gamma receptor 1 and 2. See, BioProcess International, January 2005. Table 1, "Growth factor/cytokine receptors expressed by HEK-293. Such cells will have higher protein and vector production potential and will be less dependent upon the presence of the ligand factors to be present in the medium since the cells themselves will be producing the factors and secreting them into the medium.

For other cell types, such as CHO cells, other receptor-ligand combinations may be important. For example the insulin growth factor receptor I, insulin growth factor and insulin are thought to have anti-apoptotic activity in cells. A plurality Lentiviral vectors could be constructed so that the insulin growth factor receptor (I or II), insulin growth factor (I or II), insulin and the target protein for production are all contained in the vector for transduction of production cells, such as CHO cells, and an appropriate clone selected, preferably using high-thoughput methods, to select the clone showing very high production of the target protein. The optimal clone may not be a cell that highly expresses all the engineered genes or inhibitors of gene expression, rather an optimal expression level of each of the genes, which for some may be a low level of expression. The value of the Lentiviral vector system and using a plurality of Lentiviral vectors to engineer such cell lines is that there is a random or stochastic distribution of each vector copy number in the population of cells transduced with the Lentiviral vector mixture, and therefore, by varying the amount of each vector in the mixture, the number of copies of each individual second gene or inhibitory sequence can be optimized. A preferred combination of vectors and secondary gene or gene inhibitory sequences is that each Lentiviral vector expresses the protein of interest for production and optionally in addition, at least one RNAi or gene that further promotes protein yield, or vector yield, either directly, or indirectly by affecting the viability or some aspect of the producing cell. However, it may also be beneficial to have at least one Lentiviral vector that only expresses the secondary genes or inhibitors of gene expression in order to increase the effect of these secondary sequences.

Other genes (or inhibitors of those genes) that can be engineered into Lentiviral vectors to positively effect the insulin growth factor receptor pathway, cell growth and viability are: Akt gene family members (Akt 1, Akt 2, Akt 3), p13K, Ras, Raf, MEK, MAPK p42, MAPK p44, 14-3-3 protein, Bad, and Grb/SOS. To stimulate the relevant pathways, ligands that bind to the appropriate receptors of these pathways could be expressed from Lentiviral vectors to provide the appropriate signal to the cell to positively affect protein, vector (not limited to Lentiviral vectors) or vaccine production from the cell. In some cases it may be preferred that the Lentiviral vector express both the receptor and the ligand to stimulate a particular pathway. Chimeric receptors can also be constructed to produce specific stimulation of particular pathways. This may also reduce the number of ligands that need to be produced in the cell as one ligand may stimulate a plurality of pathways through chimeric receptors that have the same ligand binding domain but different intracellular signaling domains. Conversely, chimeric receptors containing different binding domains and the same signaling domain could also be used to tailor the types of pathways that are stimulated. Chimeric receptors are known in the art and the invention can not only be used for protein, vaccine or vector production, but also for gene therapy. Other genes that can positively affect protein, vaccine or vector production in cells like CHO or 293 cells (non limiting examples) after their overexpression (or inhibition by RNAi, antisense, ribozyme, or the like) from Lentiviral vectors are bone morphogenic protein-2, PACEsol, phospholipase D PI3K (phosphoinositide 3-kinase), p70S6K (p70 S6 kinase) and ERK (extracellular-signal-regulated kinase), CDKN1, CCNB1, CDC20, CDK20, CDK4, CDKN3, CCNC, BMP1, MADH4, GA4, RCA, ATPS, HAT4, GAPDH, SP3, TCEBIL, TFAP2B, SMARCA4, EIF4E, RAB2, D1S155E, SSI-1, WT1, MYC, TSG101, SHC3, PHB, TCF12, NFIX, E2F4, TAF3C, STAT6, BCL2, NERF-2, POU2F1, NFKB1,EIF4E, BMI1, MYBL2, PIM1, KRAS2, RPA1A, JUNB, ABL1, TIM, SAS, AKT1, CSF3R, BCR, MXI1, TNFAIP6, AIP1, ILK, PTK2, CSK, CSNK2B, GK, PRKCA, MADH2, LIMK1, PIK3CA, PRKCd, PPP6C, cellular PrP, and other proteins types that are involved in growth, metabolism, cell cycling and development. A preferred embodiment is the expression of an RNAi targeted to the cellular prion protein (PrP), BSE or other adverse agent that could contaminate cell lines, in Lentiviral vector packaging or producer cells. Further preferred embodiments are a helper construct or packing cell line that expresses, as a non-limiting example, an inhibitor to cellular PrP, like an anti-PrP RNAi. Conversely, the described proteins could be either overexpressed or inhibited by RNAi, or the like, for use in gene therapy for diseases like, genetic diseases, HIV/AIDS or cancer. Preferred Lentiviral vector compositions for therapeutic use are the expression of a monoclonal antibody or a protein (or a plurality of proteins) and at least a second gene (or inhibitor of a gene, such as an RNAi) that positively affects the production of the protein in the body. The second gene or inhibitor of the gene is not limited to intracellular proteins for in vivo protein production, but could be a protein that affects the immune response, body's metabolism, hormone or cytokine production. The second gene (at least one second gene) or inhibitor of gene (at least one second inhibitor of a gene) could be produced in response to inducible promoter systems or some factor present in the body, such as a protein, virus or factor that is produced during disease. In this way, the production of the first protein or antibody (e.g., monoclonal, humanized, single-chain) can be regulated by production of the second gene. Proteins involved in correct glycosolyation of human proteins may also be expressed from a Lentiviral vector in tandem to the desired protein for production. Glycosolyation from certain species can cause undesirable effects on proteins such as monoclonal antibodies and therefore expression of an inhibitor to those enzymes that produce those specific glycosolyation patterns would increase the safety and efficacy of the recombinant protein product. For example, Glycosylation of cell lines derived from mouse and other mammals is very similar to human glycosylation. However, several significant differences might affect product quality as well as bioactivity. Most mouse-derived cell lines (e.g. NSO cells) contain an additional glycosylation enzyme. The enzyme is referred as alpha 1,3-galactosyltransferase; it mediates the transfer of Gal residues from UDPGal in alpha configuration to the internal and/or exposed Gal residues. Humans have antibodies against the alpha-Gal epitopes. Although no evidence in the literature suggests that the presence of alpha -Gal epitopes on rIgG is immunogenic to humans, regulatory agencies might express concerns about alpha -Gal residue- containing therapeutic glycoproteins. Therefore to enhance more optimal glycosolyation of proteins used form human use, an RNAi (or similar inhibitor) targeted to the mouse alpha 1,3- galactosyltransferase can be inserted into a Lentiviral vector to generate cell lines that are devoid or have reduced levels of the mouse alpha 1,3- galactosyltransferase protein so that the alpha -Gal residue is not present on therapeutic glycoproteins. Another example is CMP-N-acetylneuraminic acid hydroxylase that is present in rodent cells, such as CHO cells. This enzyme is not expressed in an active form in man and evidence suggests that the presence of Neu5Gc in recombinant therapeutic glycoproteins may elicit an immune response. Therefore, Lentiviral vectors could be engineered to contain both the protein gene of interest and reduce CMP-Neu5Ac hydroxylase activity in a Chinese Hamster Ovary (CHO) cell line, and thus the Neu5Gc content of the resulting glycoconjugates, by also containing an RNAi or antisense RNA sequence targeted to the enzyme. The two examples are not meant to be limiting, other enzymes involved in glycosolyation or other cellular processes can also be targeted - either by inhibiting unwanted enzymes/factors or by overexpressing desired enzymes to enhance or optimized the characteristics of the desired protein or factor that is to be produced.

The RNAi could also be made to potential unwanted or adventitious viruses or any virus or bacteria that would be undesirable to have replicate in the cell line used to manufacture the vector, protein, factor or vaccine. For example, the mycoplasma ribosomal or messenger RNA could be targeted by RNAi technologies to prevent mycoplasma replication and contamination. This method of inhibiting adventitious virus or bacterial replication in cells could be extended for use in the production of other viral vectors (e.g. such as adenoviral vectors, Adeno-associated viral vectors, herpes viral vectors, polyoma based vectors, retroviral vectors and Lentiviral vectors) or vaccines (e.g. such as influenza, smallpox, rubella, ebola, vaccinia). A complete set of viruses that could be the targets of such methods are found at ncbi.nlm.nih.gov/genomes/VIRUSES/viruses.html. The expression of cDNAs and RNAi in vector production systems can be used to further increase HIV vector production. For example genes that stimulate cell growth could increase cellular biosynthesis and therefore result in higher production of HIV vectors from cell lines and therefore result in higher titer vectors. Genes that could be overexpressed are those that increase carbohydrate metabolism, energy metabolism, proteins involved in the biodegradation of xenobiotics, nucleic acid and amino acid metabolism, transcription of mRNA or translation of proteins or genes that activate cell division and growth such as BcL-2, as an example. Furthermore, RNAi technology can be used to increase vector production by inhibiting genes that slow down or block cell growth, or genes that inhibit the production of HIV vector particles. For example an RNAi that are targeted to proteins that function by inhibiting cell division, cell growth, cell metabolism, nucleic acid and amino acid metabolism, transcription of mRNA or translation of proteins and therefore increase the production of HIV vector particles. A complete list of such genes and their known pathways can be found at http://www.ncbi.nlm.nih.gov/Entrez/. Several methods to increase the production of Lentiviral vectors from cell lines can be employed. First a library of cDNAs from human or another organism can be cotransfected with packaging construct(s) or inserted into a HIV vector for transduction into packaging cells containing the genes needed for production of HIV vector particles. Each step of the method can be performed in a multiwell format and automated to further increase the capacity of the system.

Another embodiment is the inclusion of an inhibitor of a gene such as an RNAi targeted to the protease gene on the Lentiviral vector in addition to the gene of interest to be expressed, or on a different Lentiviral vector but added as a mixture to the cells so that the cells are transduced with both the vector containing the gene of interest and the vector that expresses the RNAi, preferably to a protease gene or another gene that is undesirable. The protease that is to be targeted can be any single or combination of proteases that may adversely affect production or purification of the desired protein or desired factor of interest. The protein families and specific non-limiting examples are described: Cysteine proteases such as Caspases, Cathepsins; Zinc proteases (metalloproteases) such as carboxypeptidases, various matrix metalloproteases; Serine proteases such as trypsin, chymotrypsin, and elastase. The ubiquitin pathway may also be a useful target during protein production production phase in a cell line. RNAi could be inserted into Lentiviral vectors that target ubiquitin, Ubiquitin-Activating Enzyme (E1), Ubiquitin-Conjugating Enzyme (E2) and/or Ubiquitin-Protein Ligase (E3). Preferably the RNAi targeting the Ubiquitin pathway are expressed from an inducible promoter so that inhibition of Ubiquitination only occurs during a specified period of time. Induction of RNAi targeted to ubiquitin is not a limitation of the invention and it would be desirable that a Lentiviral vector constitutively express RNAi that is targeted to proteases, preferably proteases that are involved in cell death. Such proteases include but are not limited to the aspartate-specific cysteine proteases (ASCPs), serine proteases such as Omi/HtrA2, capases, the ICE family ofThiol proteases such as ICE/CED-3 proteases, granzyme B. Alternatively, the vector can express genes that inhibit apoptosis such as the IAP proteins. Such methods for modulation of cellular phenotype are not limited to protein production in cells, but can also be used in the generation of transgenic animals, and for vaccine and therapeutic purposes. A preferred embodiment for these applications is to express the second gene or gene inhibitory sequence from a tissue specific promoter.

A further preferred embodiment to any secondary gene present in a Lentiviral vector is to tag the protein with an amino acid sequence that allows for rapid removal of the secondary protein from the protein mixture that contains the desired protein for purification. In this way, any combination of proteins secondary proteins can be rapidly removed by using a single common amino acid sequence tag, allowing for rapid purification of the target protein. The target protein may have a different tag or may not have a tag at all, which is preferable if the goal is to produce and purify the native protein. Conversely, the protein of interest may be solely tagged. Also, such vectors can be used in vivo for human gene therapy and the generation of transgenic mice; and are not limited to use for in vitro systems.

### Methods of manufacturing polypeptides

The present invention also provides methods of manufacturing polypeptides utilizing lentiviral transduction vectors, such as the transduction vectors disclosed herein, and the products of such methods. The methods can comprise one or more of the following steps, e.g., transducing a host cell with a lentivirus transduction vector to form a transduced host cell, wherein said vector comprises an expressible heterologous polynucleotide coding for a heterologous polypeptide of interest; culturing said transduced host cell under conditions effective to produce said polypeptide of interest; isolating polypeptide from said host, e.g., from the culture medium when a polypeptide is secreted into the culture medium. The heterologous polynucleotide sequence coding for the polypeptide can comprise any further sequences necessary for transcription, translation, and/or secretion into the medium (e.g., secretory sequences). Any cells lines can be transduced in accordance with the present invention, including any of the cell lines mentioned herein, especially, e.g., CHO (such as CHO DG44) and HEK 293 (such as HEK293F).

Transduction vectors can be prepared routinely, including according to the methods described herein. For example, a producer cell line can be transformed with a helper plasmid (containing a suitable envelope and gag/pol precursor) and a transfer vector containing the heterologous coding sequence under conditions effective to produce functional transduction vectors. The envelope protein can be selected for its ability to transduce a target host cell in which the polypeptide is to be manufactured. For manufacturing flu vaccines the following cell lines and corresponding envelope proteins are preferred, e.g., 293 or CHO; VSV-G, ampho, Mokola, and Paramyxoviridae (for example, see the world wide web at ncbi.nlm.nih.gov/ICTVdb/Ictv/fs_param.htm).

Examples of host cells, include, e.g., mammalian cells; human cells, such A2058 melanoma, C3A liver, G-402 kidney, C8166 T-cells, Caco-2 colon, and K562 bone marrow; CHO; 293F, 293 FT, etc., including other cell lines mentioned above and below, and present on the ATCC web site (www.atcc.org ) and other sources for cells.

Any suitable or desired heterologous sequence can be expressed, including, e.g., vaccines, interferons (alpha, beta, gamma, epsilon), erythropoietin, Factor VIII, clotting factors, antibodies and fragments thereof (e.g., including single chain, Fab, and humanized), insulin, chemokines, cytokines, growth factors, angiogenesis modulatory factors, apoptosis modulatory factors, etc. Single-chain antibodies (e.g., single chain variable fragments or "scFv") can be made routinely.

In certain embodiments of the present invention, lentiviral transduction vectors can be utilized to prepare antigenic preparations that be used as vaccines. Any suitable antigen(s) can be prepared in accordance with the present invention, including antigens obtained from prions, viruses, mycobacterium, protozoa (e.g., Plasmodium falciparum (malaria)), trypanosomes, bacteria (e.g., Streptococcus, Neisseria, etc.), etc.

Host cells can be transduced with a single lentiviral vector containing one or more heterologous polynucleotide sequences, or with a plurality of lentiviral vectors, where each vector comprises the same or different heterologous polynucleotide sequence(s). For example, a multi-subunit antigen (including intracellular and cell-surface multi-subunit components) can be prepared by expressing the individual subunits on separate vectors, but infecting the same host cell with all the vectors, such that assembly occurs within the host cell.

Vaccines often contain a plurality of antigen components, e.g., derived from different proteins, and/or from different epitopic regions of the same protein. For example, a vaccine against a viral disease can comprise one or more polypeptide sequences obtained from the virus which, when administered to a host, elicit an immunogenic or protective response to viral challenge.

As mentioned, the present invention can also be utilized to prepare polypeptide multimers, e.g., where an antigenic preparation is produced which is comprised of more than one polypeptide. For instance, virus capsids can be made up of more than one polypeptide subunit. By transducing a host cell with vectors carrying different viral envelope sequences, the proteins, when expressed in the cell, can self-assemble into three-dimensional structures containing more than one protein subunit (e.g., in their native configuration). The structures can possess functional activity, including antigenic activity, enzyme activity, cell binding activity, etc. Moreover, when expressed in a suitable cell line, they can be secreted into the cell culture medium, facilitating purification. For instance, when influenza N and H capsid proteins, and optionally M protein (see below), are introduced into a production cell line using lentiviral transduction vectors, empty capsids or viral-like particles (VLP) can be formed in the cell, and then secreted into the culture media. Such VLP can be routinely isolated and purified, and then administered as an influenza vaccine. A VLP is, e.g., a self-assembled capsid which does not contain substantial amounts (e.g., is empty) ofviral RNA. A VLP is preferably able to elicit an immune response that is effective to provide at least some degree of protection against a challenge of the native infectious virus particle, or at least elicit antibodies to it.

Currently, there are many available viral vaccines, including vaccines to such diseases as measles, mumps, hepatitis (A and B), rubella, influenza, polio, smallpox, varicella, adenovirus, Japanese encephalitis, rabies, ebola, etc. The present invention can be utilized to prepare vaccines against any of the above-mentioned diseases.

The lentivirus transduction systems are of special interest because they shorten the time to develop and produce effective influenza vaccines, allowing the public health sector to respond more rapidly to changing patterns in influenza disease. Currently, influenza viruses, especially type A and B strains, are a major cause of serious illness and death around the world. In the United States, influenza ranks seventh among all causes of death, and results in high numbers of hospitalizations (200,000), work-loss days (70 million), and restricted activity days (346 million), causing significant economic impact. See, e.g., dhhs.gov/ nvpo/ influenza_vaccines.html. Influenza A viruses undergo frequent changes in their surface antigens, whereas type B influenza viruses change less frequently. Immunity following infection by one strain may not protect fully against subsequent antigenic variants. As a consequence, new vaccines against influenza must be designed each year to match the circulating strains that are most likely to cause the next epidemic. The World Health Organization has established a Global Influenza Surveillance Network which make annual recommendations on the influenza vaccine composition. The lentiviral transduction system of the present invention significantly reduces the time need to produce an effective vaccine in comparison to the standard chicken egg technology currently in use, e.g., which can take up to eight months compared to, e.g., five weeks or less using processes described herein.

Examples of viruses to which vaccines can be produced in accordance with the present invention include, e.g., orthomyxoviruses, influenza virus A (including all strains varying in their HA and NA proteins, such as (non-limiting examples) H1N1, H1N2, H2N2, H3N2, H7N7, and H3N8); influenza B, influenza C, thogoto virus (including Dhori, Batken virus, SiAR 126 virus), and isavirus (e.g., infectious salmon anemia virus). These include influenza isolated or transmitted from all species types, including isolates from invertebrates, vertebrates, mammals, humans, non-human primates, monkeys, pigs, cows, and other livestock, birds, domestic poultry such as turkeys, chickens, quail, and ducks, wild birds (including aquatic and terrestrial birds), reptiles, etc. These also include existing strains which have changed, e.g., through mutation, antigenic drift, antigenic shift, recombination, etc., especially strains which have increased virulence and/or interspecies transmission (e.g., human-to-human).

Of particular interest are influenza viruses which are panzootic and/or which cross species either because they have a broad host range, or because of recombination in the infected host, and/or because of naturally-occurring or directed mutation. For example, H5N1 (in reference to the subtypes of surface antigens present on the virus, hemagglutinin type 5 and neuraminadase type 1) is a subtype of avian influenza A, which caused an outbreak of flu in domestic birds in Asia. As of November 2005, more 120 million birds died from infection or were killed to prevent further infection from spreading. This virus has also spread into human hosts ("bird flu") where it is associated with high lethality.

An influenza antigenic preparation (such as a vaccine) can comprise one or more polypeptides that occur naturally in an influenza virion. However, it preferably does not comprise all the polypeptide genes that would give rise to the native pathogenic virus. These include, e.g., hemagglutinin (encoded by HA gene), neuraminidase (encoded by NA gene), nucleoprotein (encoded by NA gene), matrix (M1) proteins (encoded by M gene), M2 (encoded by M gene), non-structural proteins (encoded by NS gene), and polymerases. The naturally-occurring virion is sheathed in a lipid bilayer which is "studded" with integral proteins H and N ("capsid layer"). Matrix proteins (M1) form a protein layer ("matrix layer") underneath the viral membrane, and are involved in viral assembly, stability and integrity. See, e.g., Harris et al., Virol. 289:34-44, 2001. M2 protein is a membrane protein ion channel. A VLP of the present invention can comprise H, N, and optionally M1 and M2 proteins. Sequences for said proteins are known in the art and/or can be identified in GenBank. See, e.g., Widjaja et al. J. Virol., 78:8771-8779, 2004 for M1 and M2 sequences.

These can be cloned into transfer vectors, either individually or on the same plasmid, and utilized to produce transduction vectors. In one embodiment of the present invention, a plurality of transduction vectors can be prepared, each which contains a unique influenza gene sequence (e.g., coding for H, for N, and for M1 to result in a three different transduction vectors). When such vectors are co-expressed in the same host cell (e.g., CHO or 293), a self-assembling VLP is produced which can be secreted into the medium, harvested by centrifugation, and then administered as a vaccine.

Influenza A H5. At least nine subtypes of H5 have been identified. H5 infections, such as HPAI H5N1 viruses currently circulating in Asia and Europe, have been documented among humans and can cause severe illness or death.

Influenza A H7. At least nine subtypes of H7 have been identified. H7 infection in humans is rare but can occur among persons who have direct contact with infected birds. Symptoms may include conjunctivitis and/or upper respiratory symptoms. H7 viruses include, e.g., H7N2, H7N7, and H7N3), and have caused mild to severe and fatal illness in humans. The H subtypes are epidemiologically most important, as they govern the ability of the virus to bind to and enter cells, where multiplication of the virus then occurs. The N subtypes govern the release of newly formed virus from the cells.

Influenza A H9. At least nine subtypes of H9 have been identified. Influenza A H9 has rarely been reported to infect humans. However there are reports of children exhibiting flu-like syndromes when infected with H9 strains.

The present invention provides vaccines against all avian influenza subtypes (e.g., H and N subtypes), including existing subtypes, derivatives thereof, and recombinants thereof, such as subtypes and recombinants which have the ability to spread from human-to-human. Various isolates have been characterized, especially for H5 subtypes. See, e.g., Sturm-Ramirez, J. Virol., 2004, 78, 4892-4901; Guan et al., Proc. Natl. Acad. Sci., 2004, 101, 8156-8161.

Transduction vectors of the present invention can result in high levels of heterologous protein production, e.g., from about 0.1 to 0.3 mg/ml to about 5-10 mg/ml, or more, of recombinant heterologous protein per ml of unprocessed culture media, when such proteins are secreted into the culture media.

The present application also provides methods of producing antibodies. For example, methods are provided to produce monoclonal antibodies (e.g., human, mouse, and other mammalian types) without the need for hybridomas or animal models. In one non-limiting example, Lentiviral vectors expressing oncogenic proteins are transduced on peripheral blood B cells from mice previously stimulated with antigen. These vectors efficiently transduce the mouse cells to make them into antibody producing cells. In a second non limiting example, two Lentiviral vectors are engineered, one expressing the Heavy antibody chain and the second vector the light antibody chain. The constant areas of the genes are derived from the human (or other species if desired) immunoglobulin gene (eg IgG, IgM or other type of Ig). The variable areas of the genes are modified or degenerated to create diversity. The degenerate sequence can be obtained by any suitable techniques that is known in the art and cloned into the Lentiviral vector to create a library of Lentiviral vectors that express either the heavy or light immunoglobulin molecules. The antibodies can be produced by transducing cells with both vectors to produce functional antibodies that contain both heavy and light chains. Transduced and expressing cells can be selected and screened for binding to antigen, and then positive clones can be isolated and subjected to multiple rounds of affinity maturation.

An advantage of this method is that antibodies are produced in a non-biased method. Other methods, such as traditional hybridoma and Xenomouse technologies rely on B cells that have undergone clonal selection and deletion of particular antibody clones since they are reactive to endogenous, for example, mouse tissue. Some of these deleted clones may be valuable as antibodies as they could cross react with human antigens. The advantage of the described method is that there is no deletion of molecular antibody clones and they are all analyzed in a non-biased method and yet are fully humanized (if humanization is desired) antibody molecules. Another advantage of Lentiviral vectors is that the genes can be transduced into cells at high multiplicity to produce a variety of antibody type in one cell. This reduces the number of cells that need to be produced to create a library that contains a very diverse antigenic binding sites. A second advantage is placing the heavy and light genes in different Lentiviral vectors so that additional diversity can be generated by transducing cells with a higher multiplicity of infection than 1. For example, if a MOI of 10 is used for the transduction of cells with each heavy and light chain expressing Lentiviral vector, then the number of combinations of antibodies produced in each cell is 100. Therefore in a 96-well plate, where there are about 10,000 cells in a single well, the number of possible variants that can be generated with this method is 1,000,000 in a single well of a 96-well plate. Therefore, with scale, a large number of antibody variants can be generated with this method. The method does not limit to using a MOI of 10 for eachconstruct per cell, higher MOIs can also be used, as needed. For example, if a MOI of 100 is used then each cell can produce 10,000 variant antibodies and each well of a 96 well plate can produce 10,000,000,000 variants. Therefore each 96 well plate can produce 1x10¹² variant antibody molecules that can be used for screening against a target antigen, for which there are many methods known in the art (eg ELISA). Once a particular well has been identified that produces the desired antibody reaction, then the cells can be cloned by limiting dilution to find the cell clone that expresses the correct antibody. Once this clone has been identified, then PCR can be used to clone out the vectors that express the heavy and light antibody chains. The vector DNA can then be transfected with helper construct(s) to produce vector. Alternatively, this clone of cells can be transfected directly with the helper construct(s) (PEI, calcium phosphate, lipotransfection, or other transfection method known in the art), to produce the variant Lentiviral vectors. The vectors that are produced can then tittered and then transduced onto cells at a lower MOI, but a larger number of cells, to isolate a clone that produces the antibody of interest. Once the clone of cell is isolated, then the antibody can be produced to higher titers by transducing cells with higher multiplicity of infection, the same method is not limited to whole antibody molecules but can also be applied to single chain antibodies, antibody fragments, phage display and other antibody-like molecules, all known in the art. In addition to expressing the antibody the vector can express other genes to increase the production of the monoclonal antibody, or to increase their yield. Such genes can be oncogenes such as ras and myc, but other gems can also be used, such as anti-apoptotic genes such as Bcl-2. Furthermore, such vectors can be used to create monoclonal antibodies from B cells in the blood of animals that have been exposed to antigen. For example, B cells from mice exposed to antigen can be transformed into myeloma cells by using a combination of oncogenes or gene silencing RNA. Such genes include, e.g., Growth Factors, including, e.g., Amphiregulin,B-lymphocyte stimulator,Interleukin 16 (IL16), Thymopoietin, TRAIL, Apo-2, Pre B cell colony enhancing factor, Endothelial differentiation-related factor 1 (EDF1), Endothelial monocyte activating polypeptide II, Macrophage migration inhibitory factor MIF, Natural killer cell enhancing factor (NKEFA), Bone morphogenetic protein 8 (osteogenic protein 2), Bone morphogenic protein 6, Connective tissue growth factor (CTGF), CGI-149 protein (neuroendocrine differentiation factor), Cytokine A3 (macrophage inflammatory protein 1-alpha), Glialblastoma cell differentiation-related protein (GBDR1), Hepatoma-derived growth factor, Neuromedin U-25 precursor, any tumor gene, oncogene, proto-oncogene or cell modulating gene (which can be found at condor.bcm.tmc.edu/oncogene), Vascular endothelial growth factor (VEGF), Vascular endothelial growth factor B (VEGF-B), T-cell specific RANTES precursor, Thymic dendritic cell-derived factor 1; Receptors, such as Activin A receptor, type II (ACVR2), β-signal sequence receptor (SSR2), CD14 monocyte LPS receptor, CD36 (collagen type 1/thrombospondin receptor)-like 2, CD44R (Hermes antigen gp90 homing receptor), G protein coupled receptor 9, Chemokine C x C receptor 4, Colony stimulating factor 2 receptor β(CSF2RB), FLT-3 receptor tyrosine kinase, Similar to transient receptor potential C precursor, Killer cell lectin-like receptor subfamily B, Low density lipoprotein receptor gene, low-affinity Fc-gamma receptor IIC, MCP-1 receptor, Monocyte chemoattractant protein 1 receptor (CCR2), Nuclear receptor subfamily 4, group A, member 1, Orphan G protein-coupled receptor GPRC5D, Peroxisome proliferative activated receptor gamma, Pheromore related-receptor (rat), Vasopressin-activated calcium mobilizing putative receptor, Retinoic x receptor, Toll-like receptor 6, Transmembrane activator and CAML interactor (TACI), B cell maturation peptide (BCMA), CSF-1 receptor, Interferon (α, β and gamma) receptor 1 (IFNAR1). Pathways that can be modulated to increase antibody production include, e.g., ubiquitin/proteosome; telpmerase; FGFR3; and Mcl-1. Other genes that can be target to increase antibody production include are listed in the following tables:

| **Differential expression between myeloma and nonmyeloma cell lines** (Claudio et al. Blood, Vol. 100, Issue 6, 2175-2186, September 15, 2002) | | | |
|---|---|---|---|
| Clone identification | Gene/clone match | Rank | Unigene |
| Up-regulated | | | |
| PCL1920 | Glucose-regulated protein, 58 kDa (MGC:3178) | 1 | Hs.289101 |
| PCL0833 | Genomic DNA clone (chromosome 2 clone RP11-218L22) | 2 | |
| PCL2440 | EST from cDNA clone IMAGE:1694766 3' | 3 | Hs.134923 |
| MYE4362 | Genomic DNA clone (chromosome 14 BAC R-214N1) | 4 | |
| PCL1712 | Progesterone receptor membrane component-2 (PGRMC2) | 5 | Hs.9071 |
| PCL2089 | Hypothetical protein FLJ22332 (c2h2 type, zinc finger) | 6 | Hs.111092 |
| PCL1633 | Genomic DNA clone (BAC CTD-2022G18 from 7) | 7 | |
| PCL0849 | Multiple myeloma oncogene-1 (MUM1)/(IRF4) | 8 | Hs.82132 |
| PCL1492 | Myeloma EST PCL1492 | 9 | |
| MYE4007 | BUP protein | 10 | Hs.35660 |
| BCMA | B cell maturation protein (BCMA) | 11 | Hs.2556 |
| PCL1414 | Tumor rejection antigen-1 (TRA1) | 12 | Hs.82689 |
| PCL1515 | Weakly similar to mucin 2 precursor | 13 | Hs.20183 |
| PCL0308 | Proteasome (subunit, α type, 2) (PSMA2) | 14 | Hs.181309 |
| PCL0940 | Selenoprotein T | 15 | Hs.8148 |
| MYE2868 | Myeloma EST MYE2868 | 16 | |
| MYE2693 | Signal recognition particle 14 kD (SRP14) | 17 | Hs.180394 |
| PCL5267 | Myeloma EST PCL5267 | 18 | |
| MYE3869a | Myeloma EST MYE3869a | 19 | |
| PCL5298 | Similar to brain-specific angiogenesis inhibitor-1 (BAI-1) | 20 | |
| PCL1662 | Similar to chromosomal protein for mitotic spindle assembly | 21 | Hs.16773 |
| PCL0105 | CD138/syndecan-1 (SDC1) | 22 | Hs.82109 |
| MYE4521 | Annexin A2, lipocortin II, calpactin I | 23 | Hs.217493 |
| PCL4099 | Genomic DNA clone (BACCTA-227L24, 7q21.1-q21.2) | 24 | |
| PCL1657 | Hypothetical protein FLJ11200 | 25 | Hs.107381 |
| MYE2821 | Ribosomal protein L4 (RPL4) | 26 | Hs.286 |
| MYE4493 | DNA-binding protein CPBP | 27 | Hs.285313 |
| PCL3222 | Myeloma EST PCL3222 | 28 | |
| MYE1378a | Hypothetical protein FLJ10055 (similar to protein with WD repeat) | 29 | Hs.9398 |
| MYE2209 | Heat shock 70 kDa protein 5 | 30 | Hs.75410 |
| MYE4932 | X-box-binding protein-1 (XBP1) | 31 | Hs.149923 |
| PCL3824 | PIM-2 | 32 | Hs.80205 |
| PCL4079 | Genomic DNA clone (chromosome 5 clone CTC-504A5) | 33 | |
| PCL4441 | Carbonyl reductase-1 (CBR1) | 34 | Hs.88778 |
| Down-regulated | | | |
| PCL4897 | Laminin receptor-1 (67 kD, ribosomal protein SA) | 1 | Hs.181357 |
| PCL5225 | Myeloma EST PCL5225 | 2 | |
| PCL0639 | Myeloma EST PCL0639 | 3 | |
| MYE3255a | Ribosomal protein S2 (RPS2) | 4 | Hs.182426 |
| PCL4678 | Nucleophosmin | 5 | Hs.9614 |
| PCL2015 | Myeloma EST PCL2015 | 6 | |
| PCL3726 | Lymphocyte cytosolic protein-1 (L-plastin) | 7 | Hs.76506 |
| PCL3287 | Tumor protein, translationally controlled-1 (TPT1) | 8 | Hs.279860 |
| PCL4214 | Protein phosphatase-2, regulatory subunit B (PPP2R2A) | 9 | Hs.179574 |
| MYE5079 | Ribosomal protein S2 (RPS2) | 10 | Hs.182426 |
| PCL1818 | High-mobility group protein-1 (HMG1) | 11 | Hs.337757 |
| MYE2310 | Glyceraldehyde-3-phosphate dehydrogenase (GAPD) | 12 | Hs.169476 |
| PCL3027 | Myeloma EST PCL3027 | 13 | |
| MYE3019 | Ribosomal protein L31 (RPL31) | 14 | Hs.184014 |
| PCL1701 | Actin, γ-1 (ACTG1) | 15 | Hs.14376 |
| MYE1012 | Myeloma EST MYE1012 | 16 | |
| PCL2226 | Ribosomal protein L10 (RPL10) | 17 | Hs.29797 |
| MYE2056 | Ribosomal protein L5 (RPL5) | 18 | Hs.180946 |

| Clone | Sequence. | Homology to known protein or domain | Accession no. |
|---|---|---|---|
| MYE4005 | 522 | SH2 domain-containing adaptor | NM_032855.1 |
| MYE3305 | 523 | DEAD box helicases | AAC27435.1 |
| MYE6227 | 246 | TorsinB and torsinA | AAC51733.1 |
| PCL1515 | 251 | Weakly similar to mucin | A43932 |
| PCL5298 | 272 | Similar to brain-specific angiogenesis inhibitor-1 | BAA23647.1 |
| PCL1662 | 160 | Similar to chromosomal protein for mitotic spindle | S41044 |
| PCL2089 | 239 | Novel c2h2 type zinc finger | BC008901.1 |
| MYE1378 | 410 | Similar to Trp Asp (WD) repeat protein | XM_008266.3 |
| PCL1215 | 310 | Tigger 1 transposase | U49973 |
| PCL1952 | 235 | Testes development-related NYD-SP19 | AAK53407 |
| PCL2063 | 112 | Pm5 protein | NM_014287 |
| PCL2220 | 191 | DKFZp586D0222 similar to GTP-binding protein | AL136929.1 |
| PCL2520 | 389 | Ankyrin domain | Z70310 |
| PCL2835 | 132 | v-rel avian reticuloendotheliosis viral oncogene | XM_012000.2 |
| PCL2999 | 320 | APOBEC1 (apolipoprotein B editing protein) | AK022802 |
| PCL3405 | 401 | Gonadotropin inducible transcription repressor-2 | NM_016264.1 |
| MYE4184 | 365 | GTP-binding protein similar to RAY/RAB1C (RAYL) | XM_009956.1 |
| PCL3139 | 375 | ZNF140-like protein | AF155656 |
| PCL0758 | 294 | Similar to KIAA0790 (52%) | AB018333 |
| MYE1302 | 410 | PARP domain containing protein DKFZp566D244.1 | CAB59261.1 |
| MYE2885 | 183 | Hypothetical protein DKFZp434H132 | XM_007645.3 |
| MYE5546 | 347 | S68401 (cattle) glucose-induced gene (HS1119D91) | XM_009498.1 |
| MYE6872 | 220 | Hypothetical protein similar to transcription regulator | AL117513 |
| MYE5259 | 218 | Hypothetical protein DKFZP564C186 similar to Rad4 | CAB43240 |
| MYE6738 | 333 | SH3 domain-containing protein | BC008374.1 |
| PCL0791 | 235 | Plekstrin homology and FYVE zinc finger domains | XM_016836.1 |
| MYE4229a | 310 | FL20273 protein containing RNA recognition motif | NM_019027.1 |
| MYE4229a | 310 | FL20273 protein containing RNA recognition motif | NM_019027.1 |
| Cluster 96 | 707 | Novel protein disulfide isomerase | BC001199.1 |
| PCL1850 | 215 | Protein containing Myb-like DNA-binding domain | NM_022365.1 |
| PCL2185 | 138 | FLJ13660 similar to CDK5 activator-binding protein | XM_017042.1 |
| PCL4352 | 376 | FLJ11021 similar to splicing factor arginine/serine-rich-4 | XM_016227.1 |
| MYE4184 | 365 | GTP-binding protein similar to RAY/RAB1C (RAYL) | XM_009956.1 |
| PCL5805 | 210 | BH3 domain containing protein | XM_002214.1 |
| MYE4482 | 271 | MMTV receptor variant-2 (Mtvr2) | AF052151.1 |
| MYE5150 | 132 | Similar to progesterone receptor-associated p48 | XM_010011.4 |
| PCL1756 | 340 | Transient receptor potential C precursor (GIP-like protein) | P36951 |
| PCL1178 | 286 | SAM domain-containing protein FLJ21610 | XM_015753.1 |

### Methods of manufacturing lentiviral transduction vectors

The present invention also provides methods to concentrate and purify a lentiviral vector using flow-through ultracentrifugation and high-speed centrifugation, and tangential flow filtration. Flow through ultracentrifugation has been used in the past for the purification of RNA tumor viruses (Toplin et al, Applied Microbiology 15:582-589, 1967; Burger et al., Journal of the National Cancer Institute 45: 499-503, 1970). The present invention provides the use of flow-through ultracentrifugation for the purification of Lentiviral vectors. This method can comprise one or more of the following steps. For example, a lentiviral vector can be produced from cells using a cell factory or bioreactor system. A transient transfection system (see above) can be used or packaging or producer cell lines can also similarly be used. A pre-clarification step prior to loading the material into the ultracentrifuge could be used if desired. Flow-through ultracentrifugation can be performed using continuous flow or batch sedimentation. The materials used for sedimentation are, e.g.: Cesium chloride, potassium tartrate and potassium bromide, which create high densities with low viscosity although they are all corrosive. CsCl is frequently used for process development as a high degree of purity can be achieved due to the wide density gradient that can be created (1.0 to 1.9 g/cm³). Potassium bromide can be used at high densities, but only at elevated temperatures, i.e. 25° C, which may be incompatible with stability of some proteins. Sucrose is widely used due to being inexpensive, non-toxic and can form a gradient suitable for separation of most proteins, sub-cellular fractions and whole cells. Typically the maximum density is about 1.3 g/cm³. The osmotic potential of sucrose can be toxic to cells in which case a complex gradient material can be used, e.g. Nycodenz. A gradient can be used with 1 or more steps in the gradient. A preferred embodiment is to use a step sucrose gradient. The volume of material can is preferably from 0.5 liters to over 200 liters per run. The flow rate speed is preferably from 5 to over 25 liters per hour. The preferred operating speed is between 25,000 and 40,500 rpm producing a force of up to 122,000x g. The rotor can be unloaded statically in desired volume fractions. A preferred embodiment is to unload the centrifuged material in 100ml fractions. The isolated fraction containing the purified and concentrated Lentiviral vector can then be exchanged in a desired buffer using gd filtration or size exclusion chromatography. Anionic or cationic exchange chromatography could also be used as an alternate or additional method for buffer exchange or further purification. In addition, Tangential Flow Filtration can also be used for buffer exchange and final formulation if required. Tangential Flow Filtration (TFF) can also be used as an alternative step to ultra or high speed centrifugation, where a two step TFF procedure would be implemented. The first step would reduce the volume of the vector supernatant, while the second step would be used for buffer exchange, final formulation and some further concentration of the material. The TFF membrane should have a membrane size of between 100 and 500 kilodaltons, where the first TFF step should have a preferable membrane size of 500 kilodaltons, while the second TFF should have a preferable membrane size of between 300 to 500 kilodaltons. The final buffer should contain materials that allow the vector to be stored for long term storage.

The present invention also provides methods for the concentration and purification of lentiviral vectors. The method uses either cell factories that contains adherent cells, or a bioreactor that contains suspension cells that are either transfected or transduced with the vector and helper constructs to produce lentiviral vector. Non limiting examples or bioreactors, include the Wave bioreactor system and the Xcellerex bioreactors. Both are disposable systems. However non-disposable systems can also be used. The constructs can be those described herein, as well as other lentiviral transduction vectors. Alternatively the cell line can be engineered to produce Lentiviral vector without the need for transduction or transfection. After transfection, the lentiviral vector can be harvested and filtered to remove particulates and then is centrifuged using continuous flow high speed or ultra centrifugation. A preferred embodiment is to use a high speed continuous flow device like the JCF-A zonal and continuous flow rotor with a high speed centrifuge. Also preferably is the use of Contifuge Stratus centrifuge for medium scale Lentiviral vector production. Also preferably is any continuous flow centrifuge where the speed of centrifugation is greater than 5,000xg RCF and less than 26,000x g RCF. Preferably, the continuous flow centrifugal force is about 10,500x g to 23,500 x g RCF with a spin time of between 20 hours and 4 hours, with longer centrifugal times being used with slower centrifugal force. The lentiviral vector can be centrifuged on a cushion of more dense material (a non limiting example is sucrose but other reagents can be used to form the cushion and these are well known in the art) so that the Lentiviral vector does not form aggregates that are not filterable, as is the problem with straight centrifugation of the vector that results in a viral vector pellet. Continuous flow centrifugation onto a cushion allows the vector to avoid large aggregate formation, yet allows the vector to be concentrated to high levels from large volumes of transfected material that produces the Lentiviral vector. In addition, a second less-dense layer of sucrose can be used to band the Lentiviral vector preparation. The flow rate for the continuous flow centrifuge is preferably between 1 and 100ml per minute, but higher and lower flow rates can also be used. The flow rate is adjusted to provide ample time for the vector to enter the core of the centrifuge without significant amounts of vector being lost due to the high flow rate. If a higher flow rate is desired, then the material flowing out of the continuous flow centrifuge can be re-circulated and passed through the centrifuge a second time. After the virus is concentrated using continuous flow centrifugation, the vector can be further concentrated using Tangential Flow Filtration (TFF), or the TFF system can be simply used for buffer exchange. A non-limiting example of a TFF system is the Xampler cartridge system that is produced by GE-Healthcare. Preferred cartridges are those with a MW cut-off of 500,000 MW or less. Preferably a cartridge is used with a MW cut-off of 300,000 MW. A cartridge of 100,000MW cut-off can also be used. For larger volumes, larger cartridges can be used and it will be easy for those in the art to find the right TFF system for this final buffer exchange and/or concentration step prior to final fill of the vector preparation. The final fill preparation may contain factors that stabilize the vector - sugars are generally used and are known in the art.

### Vaccines and HIV therapy

Tumor cells are known to express tumor-specific antigens on the cell surface. These antigens are believed to be poorly immunogenic, largely because they represent gene products of oncogenes or other cellular genes which are normally present in the host and are therefore not clearly recognized as non-self. Although numerous investigators have tried to target immune responses against epitopes from various tumor specific antigens, none have been successful in eliciting adequate tumor immunity in vivo. Over the past 30 years, literally thousands of patients have been administered tumor cell antigens as vaccine preparations, but the results of these trials have demonstrated that tumor cell immunization has failed to provide a rational basis for the design or construction of effective vaccines. Even where patients express tumor-specific antibodies or cytotoxic T-cells, this immune response does not correlate with a suppression of the associated disease. This failure of the immune system to protect the host may be due to expression of tumor antigens that are poorly immunogenic or to heterologous expression of specific antigens by various tumor cells. The appropriate presentation of tumor antigens in order to elicit an immune response effective in inhibiting tumor growth remains a central issue in the development of an effective cancer vaccine. Also, the quantity and duration of antigen expression is also important where non-Lentiviral vectors tend not to optimize this expression. There remains a great need for a method of presenting tumor antigens, which are known to be poorly immunogenic, "self" antigens to a subject's immune system in a manner that elicits an immune response powerful enough to inhibit the growth of tumor cells in the subject. This invention overcomes the previous limitations and shortcomings in the art by providing a fusion protein comprising a chemokine and a tumor antigen which can produce an in vivo immune response, resulting in the inhibition of tumor cells. This invention also overcomes previous shortcomings in the field of HIV vaccine development by providing a fusion protein comprising a chemokine and an HIV antigen which is effective as a vaccine for treating or preventing HIV infection. Also provided are methods for to construct safer Lentiviral vectors, methods for purification of Lentiviral vectors and novel methods to used Lentiviral vectors for detection of protein-protein interactions.

The present invention also provides methods of treating or preventing HIV infection in a subject, comprising administering to the subject any combination of the following peptides derived from the following proteins: chemokine, suicide gene, HIV protein, cytokine, cell surface protein, tumor antigen, or any cellular gene that affects the production of HIV from the cell (either by overexpressing the cellular gene or inhibiting its expression by RNAi, or the like), all provided and expressed from a Lentiviral vector.

Another preferred embodiment is a Lentiviral vector for therapeutic us is that which expresses a native or fusion polypeptide comprising of any individual or combination of a human chemokine and a viral or bacterial antigen (e.g. HIV, diphtheria toxin antigen), a chemokine (e.g. IP-10, MCP-1, MCP-2, MCP-3, MCP-4, MIP 1, RANTES, SDF-1, MIG and/or MDC) or a pro-apoptotic protein, a suicide gene protein or a protein that promotes the inflammatory response.

In addition, the present invention provides a method of producing an immune response in a subject, comprising administering to the subject any of the individual or fusion polypeptides of this invention, comprising a chemokine and a human immunodeficiency virus (HIV) antigen, or a chemokine, a pro-apoptotic gene, a suicide gene and a tumor antigen, either as a protein or a nucleic acid encoding the individual or fusion polypeptide expressed from a Lentiviral vector. Also provided is a method of treating a cancer in a subject comprising administering to the subject with a Lentiviral vector expressing any of the individual or fusion polypeptides of this invention, comprising a chemokine and a tumor antigen, either as a protein or a nucleic acid encoding the fusion polypeptide.

Further provided is a method of treating or preventing HIV infection in a subject, comprising administering to the subject any combination of the following peptides derived from the following proteins: chemokine, suicide gene, HIV protein, cytokine, cell surface protein, tumor antigen, or any cellular gene that affects the production of HIV from the cell (either by overexpressing the cellular gene or inhibiting its expression by RNAi, or the like), all provided and expressed from a Lentiviral vector.

The present invention also provides an HIV vector is capable of producing HIV particles when HIV vector cells are infected with an infectious or defective HIV particle found in the body of a HIV infected individual. The vector contains an sequence that inhibits or overexpresses the following native or a mutant version of cellular host factors that results in a viral particle that is less pathogenic, or preferably non-pathogenic, than the wild-type HIV particle. These include, e.g., APOBEC family members (APOBEC 1, 2, 3A, 3B, 3C, 3D, 3E, 3F, CEM15/Apobec-3G), AID, ACF, Tsg101, Vps 4, Vps 28, Vps 37, Vps 32, ESCRT-1, ESCRT-2, ESCRT-3, TRBP-1, Sam68, proteins that contain KH domains, cellular proteins involved in dimerization and maturation of the viral particle, Hck, intercellular cell adhesion molecules (ICAMs) such as ICAM-1, ICAM-2, ICAM- 3, ICAM-4 and ICAM-5; leukocyte function-associated antigen-1 (LFA-1) and macrophage antigen 1 (Mac-1), Trim5-alpha, Trim1, human CRM1, cellular prion protein (PrP), E2F-4, cyclophillin A, members or the JAK/STAT pathway, TIP30, human Rev-interacting protein (hRIP), glycosyl-phosphatidylinositol (GPI)-anchored proteins, CD4, CD36, PRP4, HSP27, HSP70, p38 MAPK, any member of the mitogen-activated protein (MAP) kinase superfamily, Tip110, TGFbeta-1, MCP-1, Interferon regulatory factors (IRFs), IRF-1, IRF-2, IRF-3, IRF-4, IRF-5, IRF-6, IRF-7; RA5, SDF-1alpha, CCR5, CXCR4, TNF receptor superfamily (TNFRSF), CD40 ligand (CD40L, also called CD 154 or TNFSF5), IL-1, IL-2, IL-3, IL-4, IL-5, IL-6, IL-7, IL-9, IL-11, IL-13, IL-14, IL-15, G-CSF, GM-CSF, M-CSF, TNF-alpha, erythropoietin, thrombopoietin, stem cell factor, flk2/flt3 ligand and heterogenous ribonucleoprotein A2. The Lentiviral vector can include any combination of the genes or inhibitors of gene expression discussed elsewhere in this provisional patent application. A preferred combination of genes expressed in Lentiviral vector is IFN-alpha and IFN-beta. A further preferred combination is a Lentiviral vector expressing an IFN-alpha and IFN-beta separated by a IRES element or frameshift mutation that allows for translation of both genes from the same mRNA.

### Methods of eliminating cells

The present invention also provides methods of eliminating (e.g., purging) cells (e.g., in vivo or in vitro) utilizing lentiviral vectors. Such lentiviral vectors can comprise cytotoxic, cytostatic, or suicide genes that, when expressed in a target cell, lead to cell death.

For example, the present invention provides a Lentiviral vector that selectively infects and integrates into tumor cells rather than in normal cells, particularly hematopoetic stem cells that are very difficult to transduce with any vector, including a Lentiviral vector. In fact, efficient transduction of Hematopoetic stem cells to a greater than 85% efficiency could only be achieved with multiple transduction in the presence of specific stem cell factors (Davis et al Blood 2004). A greater than 90% transduction of T cells could only be achieved after stimulation of T cells with specific factors (Humeau et al 2004). Therefore, the invention uses Lentiviral vectors to selectively deliver genes into tumor cells rather than normal cells to purge hematopoietic cell (and other cell) grafts of Tumor cells, decreasing the probability of recurrent disease. The gene can be a "suicide gene", a gene that induces cellular apoptosis or a gene that stimulates the immune response. Alternatively, the gene or coding sequence may be selected whose Products offer a conditional killing mechanism for dividing cells. In this manner, the expression of a particular protein followed by the subsequent treatment is effective in killing the neoplastic cells. The subsequent treatment comprises chemical and physical treatments. Agents for chemical treatments comprise the use of enzymes or other compounds which react with the gene product to kill the host cell. Physical treatments comprise subjection of the cells to radiation, UV light, and the like. The method specifically uses a Lentiviral vector that expresses a gene of interest that is capable of purging or stimulating an immune response against contaminating cells (including without restriction, cells or a tumor or malignant, pre-malignant, proto-oncogenic, oncogenic or any abnormal cell type that may be contaminating the preparation or has the potential to provide an adverse event) by which method comprises (1) adding the vector to the cell preparation to be purged of the contaminating cells for a period of time that results in over 99% of the contaminating cells being transduced with the Lentiviral vector where normal cells in the graft are transduced with the Lentiviral vector at a frequency that is less than that of the contaminating cells; and (2) administrating the cell preparation into a patient that requires the cell preparation. The cells can be alternatively washed to remove excess vector, but this is not required. The vector can additionally express the 'purging gene of interest' (GOI) that is contained in the Lentiviral vector under a promoter that is more specifically expressed in tumor cells or with cis acting sequences that promote the stability of the GOI mRNA in oncogenic cells rather than normal cells, or cis acting sequences that promote instability of the GOI mRNA in normal cells rather than in oncogenic cells. Other promoter systems can also be used in tandem, such as inducible promoter systems. An example of this is the Tetracyline inducible promoter system.

There are several types of genes that can be used for the above invention. For example, the herpes simplex virus type I (HSV-1), thymidine kinase (TK) gene offers such a conditional killing mechanism for dividing cells. The selective advantage of using HSV-1-TK derived from the fact that the enzyme has a higher affinity for certain nucleoside analogues, such as acyclovir, ganciclovir and FIAU, than mammalian TK (McLaren at al., In: Herpes Virus and Virus Chemotherapy, R. Kono, ed., pp. 57-61, Amsterdam, Elsevier (1985)). These drugs are converted to nucleotide-like precursors and incorporated into the DNA of replicating cells, thus disrupting the integrity of the genome, and ultimately leading to cell death. Several studies have successfully made use of the conditional toxicity of TK in development studies of transgenic mice (Borrelli et al., Nature 339:538-541 (1983); Heymanet al., Proc. Natl. Acad. Sci. USA 86:2698-2702 (1989)), as a selectable marker against non-homologous recombination events in cultured cells (Capecchi, M. R., Trends in Genetics 5 (3):70-76 (1989)), for killing cells harboring wild type herpes viruses (Corey and Spear, N. Engl. J. Med. 314:686-691 (1986); Corey and Spear, N. Engl. J. Med. 314:749-756 (1986)), and in selecting for herpes virus mutants lacking TK activity (Coen et al., Science 234:53-59 (1986)). Other "suicide genes" are available (eg http://www.zgene.net/technology.html) and the use of TK is not meant to be a limiting example. Apoptotic genes can also be used in combination or singularly. Examples include: TNF Ligand Family: LTA (TNF-b), LTB (LT-b), TNF (TNF-a), TNFSF4 (OX40 Ligand), TNFSF5 (CD40 Ligand), TNFSF6 (FasL), TNFSF7 (CD27 Ligand), TNFSF8 (CD30 Ligand), TNFSF9 (4-1BB Ligand), TNFSF10 (TRAIL), TNFSF11 (TRANCE), TNFSF12 (Apo3L), TNFSF13 (APRIL), TNFSF14 (HVEM-L). TNF Receptor Family: LTBR, TNFRSF1A (TNFR1), TNFRSF1B (TNFR2),TNFRSF4 (OX40), TNFRSF5 (CD40), TNFRSF6 (Fas), TNFRSF7 (CD27), TNFRSF8 (CD30), TNFRSF9 (4-1BB), TNFRSF10A (DR4), TNFRSF10B (DR5), TNFRSF10C (DcR1), TNFRSF10D (DcR2), TNFRSF12 (DR3), TNFRSF 14 (HVEM.)Bcl-2 Family: BAD, BAK1, BAX, BCL2, BCL2A1 (bfl-1), BCL2L1 (bcl-x), BCL2L11 (bim-like protein), BCL2L2 (bcl-w), BIK, BLK, BNIP3 (nip3), BOK (Mtd), HRK, MCL-1Caspase Family: CASP1, CASP2, CASP3, CASP4, CASP5, CASP6, CASP7, CASP8, CASP9, CASP10, CASP13, CASP14. IAP Family: BIRC1 (NIAP), BIRC2 (IAP2), BIRC3 (IAP1), BIRC4 (XIAP), BIRC5 (Survivin), BIRC6 (Bruce). TRAF Family: TANK (I-TRAF), TRAF1, TRAF2, TRAF3 (CRAF1), TRAF4, TRAF5, TRAF6, TRIP. CARD Family: APAF1, ASC, BCL10 (HuE10), NOD1 (CARD4), NOL3 (Nop30), RIPK2 (CARDIAC). Death Domain Family: CRADD, DAPK2, FADD,MYD88, RIPKI. Death Effector Domain Family: CASP8AP2 (FLASH), CFLAR (CASPER), FADD, LOC51283 (BAR). CIDE Domain Family: CIDEA, CIDEB, DFFA, DFFB. p53 and ATM Pathway: ATM, CHEK1 (chk1), CHEK2 (chk2, Rad53), GADD45A, MDM2, P63, RPA3, TP53 (p53).

Immunogenic or cytokine genes can also be used singularly or in combination with either suicide or apoptotic genes. Examples of such genes are: Adaptor Proteins: FADD, IRAK1, IRAK2, MYD88, NCK2, TNFAIP3, TRADD, TRAF1, TRAF2, TRAF3, TRAF4, TRAF5, TRAF6. Cell Surface Receptors: ACVR1, ACVR1B, ACVR2, ACVR2B, ACVRL1, CD28, CD3E, CD3G, CD3Z, CD69, CD80, CD86, CNR1, CTLA4, CYSLTR1, FCER1A, FCER2, FCGR3A, GPR44, HAVCR2, OPRD1, P2RX7, TLR2, TLR3, TLR4, TLR5, TLR6, TLR7, TLR8, TLR9, TLR10.Chemokine & Receptors: BLR1, CCL1, CCL2, CCL3, CCL4, CCL5, CCL7, CCL8, CCL11, CCL13, CCL15, CCL16, CCL17, CCL18, CCL19, CCL20, CCL21, CCL22, CCL23, CCL24, CCL25, CCR1, CCR2, CCR3, CCR4, CCR5, CCR6, CCR7, CCR8, CCR9, CX3CL1, CX3CR1, CXCL1, CXCL2, CXCL3, CXCL5, CXCL6, CXCL10, CXCL11, CXCL12, CXCL13, CXCR4, GPR2, SCYE1, SDF2, XCL1, XCL2, XCR1.Cytokine & Receptors: AMH, AMHR2, BMPR1A, BMPR1B, BMPR2, C19orf10 (IL27w), CER1, CSF1, CSF2, CSF3, DKFZp451J0118, FGF2, GFI1, IFNA1,IFNB1, IFNG, IGF1, IL1A, ILIB, IL1R1, IL1R2, IL2, IL2RA, IL2RB, IL2RG, IL3, IL4, IL4R, IL5, IL5RA, IL6, IL6R, IL6ST, IL7, IL8, IL8RA, IL8RB, IL9, IL9R, IL10, IL10RA, IL10RB, IL11, IL11RA, IL12A, IL12B, IL12RB1, IL12RB2, IL13, IL13RA1, IL13RA2, IL15, IL15RA, IL16, IL17, IL17R, IL18, IL18R1, IL19, IL20, KITLG, LEP, LTA, LTB, LTB4R, LTB4R2, LTBR, MIF, NPPB, PDGFB, TBX21, TDGF1, TGFA, TGFB1, TGFB1I1, TGFB2, TGFB3, TGFBI, TGFBR1, TGFBR2, TGFBR3, TH1L, TNF, TNFRSF1A, TNFRSF1B, TNFRSF7, TNFRSF8, TNFRSF9, TNFRSF11A, TNFRSF21, TNFSF4, TNFSF5, TNFSF6, TNFSF11, VEGF, ZFPM2, RNF110 (ZNF144). Signal Transduction Proteins:
CABIN1, CALM1, CALM2, CALM3, CAMK2B, CAMK4, CDC25A, CDKN1A, CDKN2B, CHUK, CSNK2A1, CSNK2B, ENG, EVI1, GSK3A, GSK3B, IKBKB, IKBKE, IKBKG, IL18BP, ITK, JAK1, JAK2, JAK3, KPNA5, KPNB3, LAG3, LAT, MADH1, MADH2, MADH3, MADH4, MADH5,MADH6, MADH7, MADH9, MAP2K4, MAP2K7, MAP3K1, MAP3K2, MAP3K7, MAP3K7IP1, MAP3K14, MAPK3, MAPK8, MAPK9, MAPK10, MAPK 14, MHC2TA, NAP4, NBL 1, NMA, NUP214, PAK1, PLAU,PPP3CB, PPP3CC, PPP3R1, PTPRC, RIPK1, SERPINE1, SLA, SOCS1, SOCS2, SOCS3, SOCS4, SOCS5, SOCS7, TBK1, TIMP1, TRPV6, TSC22, TYK2, VAV1, VAV2, VAV3, XPO5. Responsive Genes and Other Related Genes:
AGT, BAD, BCL2, BCL3, BF, C3, CHRD, CKTSF1B1, COL1A1, COL1A2,
   COL3A1, FST, HRAS, ICAM1, ICAM2, ICAM3, ICAM4, ICAM5, IGFBP3, IGSF6, ITGB5, ITGB7, IVL, MGC27165, MYF5, NCAM1, NOS2A, ORM1, PIN1, RFX1, RFX2, RFX3, RFX4, RFX5, RFXANK, RFXAP, RFXDC1, SAA1, SELE, SELL, SELPLG, SFN, TGIF, VCAM1. Transcription Factors: ATF2, CEBPB, CREB1, CREBBP, EGR1, EGR2, EGR3, ELK1,ELK3,EP300, FKBP1B, FLJ14639 (NYP45), FOS, FOSL1, FOSL2, FOXP3, GATA3, GATA4, GRLF1, ICOS, IRF1, JUN, JUNB, JUND, MAF, MAX, MEF2A, MEF2B, MEF2D, MYC, NFAT5, NFATC1, NFATC2, NFATC3, NFATC4, NFKB 1, NFKB2, NFKBIA, NFKBIB, NFKBIE, NFKBIL1, NFKBIL2, NFRKB, RAF1, REL, RELA, RELB, RUNX1, RUNX2, SP1, SP3, SRF, STAT1, STAT4, STAT6, TFCP2, YY1.

Suicide gene therapy can also be referred to as prodrug-activation gene therapy which can be used to increase the sensitivity of target cells to apoptosis induced by prodrugs. Introduction of a suicide gene using a lentiviral vector provides the tumor cell with the capacity for localized prodrug activation, restricting production of the toxic drug metabolite to the targeted tissue. Suicide gene therapy systems include, e.g., HSV-tk in combination with the antiviral prodrug ganciclovir and the bacterial gene cytosine deaminase in combination with the prodrug 5-fluorocytosine. Cytochrome P-450 enzymes can also be used, which can be combined with a variety of anticancer prodrugs, such as cyclophosphamide and its isomer ifosfamide.

There has in the past been an attempt to use vectors for the treatment of Graft vs Host ("GVH") disease, which is a side-effect of allogeneic transplantation with high mortality. These have failed because either high transduction efficiency of donor lymphocytes could not be accomplished, or the cells responsible for graft vs host disease could not be effectively targeted. This invention provides for the use of lentiviral transduction vectors to address both deficiencies. The present invention provides a new strategy for the treatment of Graft vs Host Disease (GVHD) during allogeneic transplantation. Presently, allogeneic transplantation results in a high mortality rate due to graft vs host disease where lymphocytes from the donor recognize the host as foreign and start destroying normal host tissue. While lymphocytes from the donor can destroy tumor cells effectively, the GVHD side effects prevent allogenic and unrelated donor transplantation as a means to treat various forms of cancer. The present method employs Lentiviral vectors for the treatment or prevention of graft vs host disease. The method uses a Lentiviral vector that expresses a suicide gene that is used to transduce donor lymphocyte populations.

Other strategies include the expression of apoptotic genes or RNAi to survival factors that are expressed from inducible promoters. The payloads described are non-limiting examples and any gene or gene silencing sequences can be used to modulate the function of the allogeneic T cells, rather than simply killing the cells at some point in the future. The method stimulates donor lymphocytes with anti-CD3 and anti-CD28 antibodies (or other stimulants such as mitogens, cytokines, other factors) prior to or during transduction with the Lentiviral vector expressing the suicide gene or inducible cell death gene or RNAi. Stimulation will allow for high and even complete transduction of lymphocyte populations with the Lentiviral vector. Therefore, once the transduced cells are infused into the patient, then if the allograft caused GVHD, then the GVHD can be treated with a pro-drug to induce cell killing of the lymphocytes that are mediating GVHD. The level of produg can also reduce GVHD in a dose dependent manner so that the graft vs tumor effect can be maintained.

Since it is the alloreactive T cells that are the mediators of GVHD a preferred method to treat the lymphocyte or peripherial blood cell population is to more specifically target these cells with the vector. As it is known that Lentiviral vectors more effectively transduce cells that are more activated, alloreative T cells will be more efficiently transduced with Lentiviral vectors if they are selectively activated over those T cells that are not alloreactive. Specific activation of alloreactive T cells can be accomplished by mixing donor lymphocytes (or leukocytes, or CD4 T cells) with recipient cells (either leukocytes, red cells or other recipient cells; cells can be irradiated or treated to kill or prevent cell growth) or an extract of the recipient's cells, and simultaneously add vector to the population at an appropriate MOI (multiplicity of infection) that selectively transduces the alloreactive cells and not the non-alloreactive cells that are not stimulated by the mixing of the cells. A preferred method is to mix the recipient's red blood cells with the donor lymphocytes as these cells express MHC antigens, including the minor MHC antigens (Zimring et al., Blood. 2006 Jan 1;107(1):187-9) and they are not cells that will not be transduced stably with the vector as they are enucleated. This MOI can be readily determined by those familiar in the art where a reporter expressing vector can be used to determine which cells have been transduced. After mixing of the red blood cells with the donor lymphocytes and transduction with the Lentiviral vector, the lymphocytes are washed and preferably isolated from the red blood cells prior to infusion into the patient. The separation of red blood cells from lymphocytes can be accomplished by several techniques including bead separation or ficoll gradient centrifugation and is commonly known in the art. The advantage of using isolated red blood cells over other cell types for stimulation is (1) they are readily available, (2) they are readily removed after stimulation (3) they do not grow and therefore do not contribute to sustained stimulation of donor lymphocytes and (4) they are not transduced with the vector. The transduced alloreactive cells can be destroyed either in vitro before infusion, or after infusion into the patient. The cells can also alternatively be stimulated with an cell extract or peptides that are patient specific and derived from the patient's particular minor or major histocompatibility complex (MHC) genes. Preparation of the extract or peptides/proteins that express a specific MHC gene are known in the art. Preferably the extract is derived from non-tumor tissues so that allo-specific cells are more specifically transduced than cells that are specific for antigens that are disease related. The extract or peptide/proteins are pulsed on the donor cells to stimulate the alloreactive cells to enable efficient transduction by the Lentiviral vector. After transduction with the vector, the cells can be washed and then are ready for freezing or infusion into the patient. It may be preferable to culture the cells in IL-2 for a short period of time before infusion into the patient.

An alternative method for transduction of T cells employs the use of soluble CD3, IL-2 (or a combination of two soluble factors, or a combination of one soluble and one immobilized factor or ligand) in a mixed lymphocyte population. A Lentiviral vector is added to a population of lymphocytes, and specifically not to a population of purified CD4 T cells, in the presence of soluble CD3 and IL-2. Alternatively, soluble CD3 and IL-2 can be expressed from a facilitator vector, as described elsewhere in this application. The mixed lymphocyte environment acts to stimulate the cells in addition to CD3 and IL-2 allowing for high efficiency transduction by a Lentiviral vector when it is added to the cells. This method of transduction of T cells by Lentiviral vector may be broadly utilized for a wide varietly of applications, including, but not limited to the treatment of genetic, infectious and oncogenic diseases.

Furthermore, method of optionally incorporating suicide or safety gene(s) into cells have wide applications. One non-limiting application is the combination of Lentiviral vector mediated expression of native or chimeric T cell receptors that are targeted to diseased cells in combination with suicide genes. Such genetically modified cells (which can be autologous or derived from immortalized cells) can home to disease cells, such as cancer cells or cells infected with a pathogen, and then the patient can be treated with a pro-drug to eliminate both the T cells and with a by-stander effect, kill the cancer, infected cell or diseased cell. Such an approach can be used solely or in combination with any of the other approaches described in this application.

One non-limiting example of the method employs the use of a Lentiviral vector that contains a gene that can kill or destroy the Lentiviral vector transduced cell. Preferably the gene is either expressed in an inducible manner and/or is gene that is only activated in the presence of a pro-drug. There are many inducible promoters available - non-limiting examples are the tetracycline inducible promoter or tissues specific promoters. There are many suicide genes available including the Herpes Virus Thymidine Kinase gene and the Drosophila Dm-dNK kinase gene, which sensitizes cells transduced with these genes to a pro-drug to induce cell killing or death after the drug is introduced either in vitro or in vivo. Promoter inducible gene silencing sequences can also be used to induce cell death.

The present invention also provides methods for the treatment of blood diseases by promoter specific expression of suicide genes. There are many suicide genes available including the Herpes Virus Thymidine Kinase gene and the Drosopilla Dm-dNK kinase gene, which sensitizes cells transduced with these genes to a pro-drug to induce cell killing or death after the drug is introduced either in vitro or in vivo. New methods of functional genomics have identified genes that have increased transcriptional activity or post transcriptional mRNA survival in diseased cells. These unique attributes of diseased cells can be used to develop Lentiviral vector strategies forthe treatment of these diseases. The method employs the use of a Lentiviral vector that expresses a suicide gene in a tissue specific manner. A non-limiting example is a Lentiviral vector can express the Drosophila Dm-dNK kinase gene under the control of the CD 19 B cell specific promoter for the treatment of B-cell related leukemias and lymphomas. This Lentiviral vector is delivered into stem cells by bone marrow transplantation. Upon the development of recurrent leukemic disease, the patient is given the pro-drug and all cells that express CD 19 (all B cells) will be killed. In a patient that has aggressive cancer loss of functional B-lymphocytes is tolerated and the patient can be supplemented with immunoglobulins intravenously. By killing the recurrent B-cell related tumor cells, the patient's life is saved. This strategy can be made more specific to the tumor cell type by using a promoter or post-transcriptional element that is found only in the tumor and not normal B cells.

Elimination of target cells can also be accomplished using lentiviral vectors that transduce gene cassettes into cells that comprise tissue-specific promoters operably linked to suicide, cytotoxic, and cytostatic genes. For example, hematopoietic stem cells can be transduced with a suicide gene that is specifically expressed from an endothelial cell promoter. When some of the stem cells differentiate into endothelial cells, these cells can be specifically killed by a prodrug that activates the suicide gene. Recently, it was discovered that during bone marrow transplantation for the treatment of cancer, the vascular endothelium from cancer cells are derived from bone marrow cells. So, by marking them like a Trojan horse, one can kill the endothelium tumors need to grow and form metastasis. Similarly, when stem cells are utilized therapeutically (e.g., to regenerate heart, pancreas, liver, neural, vascular, etc. tissues), undesirable transdifferentiation events can be controlled by transducing the stem cells with gene cassettes that, when expressed in the undesirable cell type, result in its death.

### Use of lentiviral vectors

Lentiviral vectors, particularly HIV vectors, can realize the potential of such systems to create a library of cells with varying phenotypes to specifically test the specificity and safety of various drugs and biologics.

Methods, and compositions for use therein, are provided for directly, rapidly and unambiguously measuring in a high throughput setting the function of sample nucleic acids of unknown function, using HIV vector, a packaging plasmid or a packaging cell line. The method includes the steps of constructing a vector in plasmid form by inserting a set of cDNAs, DNAs, ESTs, genes, synthetic oligonucleotides, shRNAi, ddRNAi or a library of nucleic acids into HIV vector plasmids that are devoid of HIV genes that are expressed as functional HIV proteins, co-transfecting the HIV vector plasmid with helper plasmid(s) in to a cell line or packaging cell line that have complementing components necessary for replication and packaging of the HIV vector. The result is to produce a set or library of recombinant HIV vectors preferably in a miniaturized, high throughput setting, including but not limited to 96 and 384 well formats, arrays, printing vectors onto slides and similar methods. To identify and assign function to product(s) encoded by the sample nucleic acids, a host or host cell is transduced in a high throughput setting with the recombinant HIV vectors which express the product(s) of the sample nucleic acids and thereby alter a phenotype of a host.

A preferred embodiment is a HIV vector containing a cDNA or RNAi library that is transfected or transduced into a cell or packaging cell line where the helper expresses an envelope gene that allows for the packaged vector particle to infect or transduce neighboring cells for vector amplification. Given that each vector initially transfected or transduced into the packaging cells or packaging cell line are identical, those vectors that are produced more efficiently will amplify more rapidly thanthose vectors that are produced not as efficiently. The vector titer in each sample can then be assayed by numerous methods. One such method is an ELISA assay, an assaywell known in the art, where the protein being assayed is the p24 antigen from HIV in the medium of the cells. Other assays that can be used to determine which clones are producing HIV vectors more efficiently is by using fluorometric methods such as the green fluorescent protein that is encoded in the vector. A preferred embodiment for use of fluorescent proteins is to express the cDNA and the fluorescent protein off the same promoter and within the same mRNA, separated by a translation initiation sequence to initiate the translation of the second gene product. Such translation initiation sequences are known in the art. For example the Internal Ribosome Entry Site (IRES) sequence is one that is commonly used. Generally, expression from the downstream gene from the IRES is not as efficient as from the upstream gene. If the level of expression of the downstream gene is lower than acceptable then a Post-transcriptional regulatory element (PRE) can be inserted distally of the downstream gene in order to increase its expression. The method can be modified to generate vector envelope proteins with modified tropisms due to the error prone reverse transcriptase molecule in HIV and the ability of HIV to recombine. During each round of amplification the HIV vector creates an error in its genome and therefore can modify the envelope sequences contained in it and therefore change the binding affinity and possibly tropism of the viral vector. By using a target cell as the packaging cell line (e.g. a particular type of cancer cell) containing helper components, the vectors with increased tropism to the said cell line and will be preferentially selected for during each round of replication, in contrast to those vectors that have decreased tropism or are defective for replication. After selection the modified envelopes can be isolated by PCR using vector specific primers located 5' and 3' to the envelope sequence, and characterized. The envelope sequence need not start with the native envelope sequence, but can consist of a library of envelope protein variants that can be generated by several techniques known in the art. The selection procedure need not be limited to cell culture.

Transgenic animals can be created with packaging components for whole animal selection of HIV vectors in the animal. The packaging component may need to be designed to be species specific; for example for replication in monkeys, SIV packaging genes (e.g. gag, pol, regulatory or accessory genes) may be preferred to HIV packaging genes, while nevertheless using the HIV genome as the transfer vector (e.g. The 5' HIV - LTR up to a portion of the non coding portion of HIV gag containing the packaging sequence, optionally the rre element and its splice acceptor sequence, the envelope gene, and the 3' HIV-LTR). Under a tissue specific promoter, the envelope gene can then be expressed in a specific organ or tissue upon administration of the vector into the animal. In this way using transgenic animals that contain certain packaging genes for packaging and mobilization of the vector can create highly specific targeted vectors.

Another embodiment is the automation of the process when determining the function of genes using a Lentiviral vector. To determine the function of genes, a set of cDNAs or RNAi is inserted into a HIV vector to create a library of HIV vectors, each expressing a cDNA, an RNAi, or a cDNA and an RNAi, two cDNAs, two cDNAs and an RNAi, a cDNA and two RNAi's, or at least two RNAi's targeted to particular genes of interest. Each step of the method can be performed in a multiwell format and automated to further increase the capacity of the system. This high throughput system facilitates expression analysis of a large number of sample nucleic acids from human and other organisms both in vitro and in vivo and is a significant improvement over other available techniques in the field. The present invention uses high-throughput generation of recombinant HIV vector libraries containing of one or more sample nucleic acids followed by high-throughput screening of the adenoviral vector libraries in a host to alter the phenotype of a host as a means of assigning a function to expression product(s) of the sample nucleic acids. Libraries of HIV vectors are generated in a high-throughput setting using nucleic acid constructs and complementary packaging cells. The sample nucleic acid libraries can be a set of distinct defined or undefined sequences or can be a pool of undefined or defined sequences. The first nucleic acid construct is a relatively small and easy to manipulate adapter plasmid and an expression cassette with the sample nucleic acids. The second nucleic acid construct contains one or more nucleic acid molecules that partially overlap with each other and/or with sequences in the first construct and contains at least all HIV vector sequences necessary for replication and packaging of a recombinant HIV not provided by the adapter plasmid or packaging constructs or cells. Co-transfection of the first and second nucleic acid constructs into the packaging cells leads to homologous recombination between overlapping sequences in the first and second nucleic acid constructs and among the second nucleic acid constructs when it is made up of more than one nucleic acid molecule. The HIV vector library is introduced into a host in a high-throughput setting which is grown to allow sufficient expression of the product(s) encoded by the sample nucleic acids to permit detection and analysis of its biological activity. The host can be cultured cells in vitro or an animal or plant model. Sufficient expression of the product(s) encoded by the sample nucleic acids alters the phenotype of the host. Using any of a variety ofin vitro and or in vivo assays for biological activity, the altered phenotype is identified and analyzed and function is thereby assigned to the product(s) of the sample nucleic acids.

There are several advantages to present invention over currently available techniques. The entire process lends itself to automation especially when implemented in a 96-well or other multi-well format. The high-throughput screening using a number of different in vitro assays provides a means of efficiently obtaining function information in a relatively short period oftime. The member(s) of the recombinant HIV vector libraries that exhibit or induce a desired phenotype in a host in vitro or in situ are identified to collapse the libraries to a manageable number of recombinant adenovirus vectors or clones which can be tested in vitro in an animal model. Another distinct advantage of the subject invention is that the methods produce Replication Competent Lentivirus (RCL)-free adenovirus libraries. RCL contamination throughout the libraries could become a major obstacle especially if libraries are continuously amplified for use in multiple screening programs.

Another embodiment is a Lentiviral vector that expresses the Glutamine synthetase (GS) gene with the intended recombinant protein or monoclonal antibody gene. It is know that GS is a very important metabolite and results in strong selection of cells that show high expression of the recombinant protein or monoclonal antibody. The HIV vector would contain the recombinant protein gene and the GS gene in the same vector. Alternatively, a plurality of vectors that contain the recombinant protein, GS or another gene that promotes the yield of the recombinant protein is also a preferred embodiment of the invention. Other selection methods can be used, including but not limited to puromycin, surface marker gene expression and other methods.

The present invention also describes a method to isolate genes to increase the production yields of a protein, a vaccine, or a monoclonal antibody using high throughput methods described above. A library of Lentiviral or HIV vectors expressing cDNAs or RNAi is constructed with either the recombinant protein or monoclonal antibody expressed on a separate Lentiviral or HIV vector or the vector containing the library of cDNAs or RNAi (including shRNAi and ddRNAi, or other inhibitors of gene expression such as ribozymes, antisense, aptamers, transdominant mutant proteins and the like). The vector is produced and added to the cells used to manufacture the protein and individual cells cloned that express the recombinant protein using a high throughput format described above. The amount of protein production can be measured by methods known in the art and clones expressing high levels of protein can be identified. The specific cDNA or RNAi from the library can be amplified using vector specific primers as described above and the sequence characterized. This cDNA or RNAi can then be used to increase the production of other proteins or monoclonal antibodies by including it in every HIV vector construct, or by constructing cell lines that now constitutively express the identified cDNA or RNAi.

Another aspect of the present invention is a Lentiviral vector that expresses an RNAi targeted to a protease gene, with the intended recombinant protein, monoclonal antibody gene or vaccine. It is know that proteases significantly decrease the yield of the intended recombinant protein or monoclonal antibody during the purification process. The HIV vector would contain the recombinant protein gene and an RNAi to one or more protease genes in the same vector. Alternatively, a plurality of vectors that contain the recombinant protein, an anti-protease RNAi or another gene that promotes the yield of the recombinant protein during the purification process is also a preferred embodiment of the invention.

The present invention also provides methods to isolate genes to increase the yields of protein or monoclonal antibody production during the downstream purification process by inhibiting proteins that affect yield during their purification. This method is very amenable to the high throughput methods described above. At least a single library of Lentiviral or HIV vectors expressing cDNAs or RNAi is constructed with either the recombinant protein or monoclonal antibody expressed on a separate Lentiviral or HIV vector or the vector containing the library of cDNAs orRNAi. The vector is produced and added to the cells used to manufacture the protein and individual cells cloned that express the recombinant protein using a high throughput format described above. The recombinant protein or monoclonal antibody is then purified and yield measured by methods known in the art. The specific cell clones containing high yielding protein or monoclonal antibody are identified. The specific cDNA or RNAi from the library can be amplified using vector specific primers as described above and the sequence characterized. This cDNA or RNAi can then be used to increase the production of other proteins or monoclonal antibodies by including it in every HIV vector construct, or by constructing cell lines that now constitutively express the identified cDNA or RNAi.

An embodiment is also a Lentiviral vector that expresses and cDNA or an RNAi that inhibits a potential viral, prion or bacterial contaminant of the cell line that is producing the monoclonal antibody, protein or vaccine. One non-limiting example is an RNAi that is expressed in the protein expression Lentiviral vector and is targeted to the Bovine Spongiform Encephalopathy agent, or the Creutzfeld-Jakob Disease (CJD) agent, a potential contaminant of preparations during the manufacture of biologics. Expression of the anti-BSE or anti-CJD RNAi will minimize the risk for contamination of the preparation by the BSE or CJD agent and therefore increase the safety of such engineered biologic preparations. The HIV vector would contain the recombinant protein gene and an RNAi to one or more agents that are of concern for contamination. Alternatively, a plurality of vectors that contain the recombinant protein, an anti-agent RNAi or another gene that inhibits the replication of the agent also a preferred embodiment of the invention. The invention can also be modified to include a gene or RNAi to minimize the production of any gene that is considered deleterious or adverse to the production and quality of the recombinant product.

Lentiviral vectors can also be used to generate a library of cell lines that differ in the over-expression or inhibition of one or a plurality of genes. A plurality of vectors expressing genes is added to the cells in order to obtain a desired cell with a specific phenotype. The genes can be cloned upstream from a fluorescent marker gene using elements such as the IRES element, described above as an example, so that the marker and gene-of-interest can be translated from the same mRNA. The cells are cloned, preferably by high-throughput methods described above, and the cells with the correct combination of genes over-expressed and other genes down-regulated by RNAi mediated inhibition. One of the preferred genes could be a gene that immortalizes the cell, if the starting material is a primary cell, such as the expression of telomerase reverse transcriptase (TERT), or other methods as described in patents (US6686159 or 6358739). However, any cell, including existing cell lines can be used as starting material.

Another exemplary embodiment is the genetic modification of cells with a plurality of Lentiviral vectors comprising of expressed genes of interest and/or inhibitors of gene expression, and then cell clones are isolated using high throughput methods to isolate a clone of cells with a desired genotype and/or phenotype.

The present invention also provides methods of identifying a test compound as selectively affecting a gene of interest or its expression products or downstream genes or proteins in its pathway comprising of culturing a plurality of Lentiviral vectors with cells to genetically modify them to contain both a gene that overexpresses a gene of interest and; either overexpresses at least a second gene, or at least an inhibitor sequence for a second gene of interest, wherein the plurality of cells are then isolated by high throughput methods to isolate a clone of cells with a desired genotype and/or phenotype.

The present invention also provides method of identifying an agent that alters the level of protein or gene expression in a mammalian cell where the method comprises genetically modifying a cell population with a plurality of Lentiviral vectors with cells to genetically modify them to contain both a gene that overexpresses a gene of interest and; either overexpresses at least a second gene, or at least an inhibitor sequence for a second gene of interest, wherein the plurality ofcells are then cloned to isolate a clone of cells with a desired genotype or phenotype; and then incubating said cells in the presence of a candidate agent and determining the effects of the said candidate agent on the cells.

Another aspect of the present invention is a a Lentiviral vector that expresses a cDNA or an RNAi that stimulates the immune response. A preferred embodiment is a HIV vector that expresses GM-CSF, CD40L and/or any cytokine or stimulant of the immune response. The vector can be one that mobilizes or a vector that does not mobilize, depending upon the desired intention for treatment or vaccination. In addition to the cytokine gene, a suicide gene can be inserted into the vector to induce apoptosis in cells containing the vector after administration of a prodrug.

Another embodiment is the use of a Lentiviral vector for the discovery of novel protein-protein interactions in mammalian cells using two-hybrid technology. One example is provided by the Promega Corporation (www.promega.com). Two-hybrid systems are extremely powerful methods for detecting protein:protein interactions in vivo. The basis of two-hybrid systems is the modular domains found in some transcription factors. In the CheckMate™ Mammalian Two-Hybrid System, the pBIND Vector contains the yeast GAL4 DNA binding domain upstream of a multiple cloning region, and the pACT Vector contains the herpes simplex virus VP16 activation domain upstream of a multiple cloning region. In addition, the pBIND Vector expresses the Renilla reniformis luciferase, which allows the user to normalize the transfection efficiency. The two genes encoding the two potentially interactive proteins of interest are cloned into pBIND and pACT Vectors to generate fusion proteins with the DNA binding domain of GAL4 and the activation domain of VP16, respectively. The pG5luc Vector contains five GAL4 binding sites upstream of a minimal TATA box, which in turn, is upstream of the firefly luciferase gene (luc+). The pGAL4 and pVP16 fusion constructs are transfected along with pG5luc Vector into mammalian cells. Two to three days after transfection, the cells are lysed, and the amounts of Renilla luciferase and firefly luciferase are quantitated using the Dual-Luciferase® Reporter Assay System. Interaction between the two test proteins, as GAL4 and VP16 fusion constructs, results in an increase in firefly luciferase expression over the negative controls. Such a Two hybrid system could easily be adapted into a Lentiviral vector for direct screening of protein-protein interactions in mammalian cells.

The topic headings set forth above are meant as guidance where certain information can be found in the application, but are not intended to be the only source in the application where information on such topic can be found. The entire disclosure of all applications, patents and publications, cited above are hereby incorporated by reference in their entirety. U.S. Provisional Application Nos. 60/653,386, filed February 16,2005; 60/660,310, filed March 10, 2005; 60/682,059, filed May 18, 2005; and 60/723,768, filed October 5, 2005, are hereby incorporated by reference in their entirety.

### Aspects of the invention

1. A lentiviral helper plasmid comprising:
   a) lentivirus 5' LTR comprising a functional native promoter operably linked to a polynucleotide sequence coding for lentivirus gag and pol, and a heterologous polyA signal which is effective to terminate transcription driven by said native promoter;
   b) heterologous promoter operably linked to an envelope coding sequence, and a heterologous polyA signal which is effective to terminate transcription driven by said heterologous promoter,
      wherein said native and heterologous promoters are present in said plasmid in opposite transcriptional orientations, and said plasmid is lacking a functional packaging sequence.
2. A lentiviral helper plasmid of aspect 1, wherein said plasmid comprises a TAR element which is obtained from a different lentiviral species than the 5' LTR and comprises an RRE element which is obtained from a different lentiviral species than the 5' LTR.
4. A lentiviral helper plasmid of aspect 1, wherein said 5' LTR is native.
5. A lentiviral helper plasmid of aspect 1, wherein said plasmid further comprises an expressible polynucleotide sequence coding for Tat polypeptide or Rev polypeptide which is operably linked to a promoter.
6. A lentiviral helper plasmid of aspect 1, wherein said 5' LTR is HIV-1 or HIV-2.
7. A lentiviral helper plasmid of aspect 1, wherein said polynucleotide sequence coding for lentiviral gag and pol is HIV-1 gag and pol or HIV-2 gag and pol.
8. A lentiviral helper plasmid of aspect 1, wherein the polynucleotide sequence coding for gag and pol comprises at least one non-naturally occurring codon to improve translation of said coding sequence when expressed in a compatible host.
9. A lentiviral helper plasmid of aspect 1, wherein a polynucleotide sequence is present between the pol and envelope coding sequences, which is a termination codon, or, a p7 KETWETWWTE coding sequence.
10. A lentiviral helper plasmid of aspect 1, wherein said envelope coding sequence is for VSV-G envelope or a filovirus envelope.
11. A lentiviral helper plasmid of aspect 1, further comprising an anti-sense polynucleotide that is effective to inhibit translation of said envelope coding sequence.
12. A lentiviral transfer vector comprising:
   a) lentivirus 5' LTR;
   b) lentiviral packaging sequence distal to said 5' LTR;
   c) modified lentivirus 3'LTR that comprises TATA box sequence, but is lacking 3' U3 sequences 5' to the said TATA box sequences, wherein said 3' LTR has reduced transcription activity.
13. A lentiviral transfer vector of aspect 12, further comprising d) heterologous promoter operably linked to a heterologous polynucleotide sequence.
14. A lentiviral transfer vector of aspect 12, wherein the lacking 3' U3 sequences are 5' to within 20 nucleotides of the TATA box sequences.
15. A lentiviral transfer vector of aspect 12, wherein the 3'LTR further comprises a second heterologous promoter operably linked to a second heterologous polynucleotide sequence, wherein said promoter and heterologous polynucleotide sequence are inserted into the 3 ' LTR in a position which is effective to reduce the transcription activity of said 3' LTR.
16. A lentiviral transfer vector of aspect 12, further comprising a second heterologous promoter operably linked to a heterologous sequence coding for a second gene of interest.
17. A lentiviral transfer vector of aspect 16, wherein said first and second heterologous coding sequences are separated by an internal ribosome entry site.
18. A lentiviral transfer vector of aspect 16, wherein each of said heterologous coding sequences further comprise a heterologous polyA signal which is effective to terminate transcription driven by said promoters.
19. A lentivirus packaging system for producing a lentivirus transducing vector, comprising
   a) a lentiviral helper plasmid of aspect 1
   b) a lentiviral transfer vector of aspect 12, and
   c) a plasmid comprising a coding sequence for a rev polypeptide operably linked to a heterologous promoter, and a coding sequence for a tat polypeptide operably linked to a heterologous promoter.
20. An isolated cell comprising the helper vector of aspect 1.
21. An isolated cell comprising the transfer vector of aspect 12.
22. An isolated cell comprising the lentivirus packaging system of aspect 19.
23. A method for producing a lentiviral transduction vector, comprising
   co-expressing the plasmids comprising the packaging system of aspect 19 in a host cell under conditions effective to produce a transduction vector.
24. A method for manufacturing a polypeptide of interest in a host cell, comprising
   transducing a host cell with a lentivirus transduction vector to form a transduced host cell, wherein said vector comprises an expressible heterologous polynucleotide coding for a secreted heterologous polypeptide of interest.
25. A method of aspect 24, wherein said host cell is a CHO or a 293 cell.
26. A method of aspect 24, further comprising culturing said transduced host cell under conditions effective to produce said polypeptide of interest.
27. A method of aspect 24, wherein said host cell is transduced with a plurality of lentivirus transduction vectors, wherein each vector comprises a different heterologous polynucleotide coding for a different polypeptide.
28. A method of aspect 27, wherein each of said heterologous polynucleotide codes for at least one capsid polypeptide of a virus capsid, which when expressed in said host cell, self-assemble into said viral capsid.
29. A method of aspect 28, wherein at least one polynucleotide codes for a hemagglutinin or a neuraminadase polypeptide of an influenza virus.
30. A method of aspect 24, wherein said host cell is transduced with polynucleotides coding for hemagglutinin, neuraminadase, and matrix (M1) polypeptides.
31. A method of aspect 30, wherein each polynucleotide is present in a different viral transduction vector.
32. A product of aspect 30.
33. A method for identifying polypeptides or genes which improve the manufacture of polypeptides in host cells, comprising:
   producing a plurality of transduced host cells, each cell being transduced with at least two different lentivirus transduction comprising an expressible heterologous polynucleotides that differ from each other in their sequence, and
   screening said host cells for a functionality activity associated with the heterologous sequence.
34. A method of aspect 33, wherein said heterologous sequence is a RNAi sequence, a coding sequence for a polypeptide, or anti-sense to a gene of interest.
30. In a lentiviral transduction vector, the improvement comprising a heterologous polynucleotide inserted into a 3' LTR, where such insertion results in a 3' LTR with minimal transcriptional activity.
35. A method of treating GVHD disease associated with transplantation of donor lymphocytes into a host, comprising
   transducing donor lymphocytes with a lentiviral transduction vector comprising an expressible or selectively expressible polynucleotide sequence that encodes a cytostatic or cytotoxic element,
   optionally, transducing the cells in the presence of recipient polypeptides or cells,
   and
   infusing said transduced lymphocytes into said host.
36. A method of aspect 35, wherein said selectively expressible gene is operably linked to an inducible promoter or a promoter that is activated in the presence of an exogenously introduced chemical.
37. A method of aspect 35, wherein said selectively expressible gene codes for an RNAi or pro-apoptotic polypeptide.
38. A method of aspect 35, wherein said cytotoxic element is a coding sequence for herpes thymidine kinase or a multisubstrate kinase gene.
39. A method of aspect 38, further comprising administering an effective amount of ganciclovir, AZT, flurada(® or acylovir, wherein said amount is effective to result in the cell death of said transduced host cell.
40. A method of aspect 35, further comprising contacting said donors cells with an effective amount of a host self-antigen at the same time or prior to transducing said donors cells.
41. An expression vector, comprising:
   a) lentivirus 5' LTR comprising a functional native promoter operably linked to a polynucleotide sequence coding for a native lentivirus gag and pol, and a heterologous polyA signal which is effective to terminate transcription driven by said native promoter, wherein a translation termination signal is present downstream of the start of the gag-pol sequence,
   b) a splice acceptor site located downstream of the gag-pol sequences and
   c) a heterologous polynucleotide sequence located downstream to the gag-pol sequence that is operably linked to the 5 'LTR promoter.
42. A lentiviral transduction vector comprising a T cell receptor and a cytotoxic element.
43. A lentiviral vector packaging or producer cell line that expresses an inducible gene inhibitory or silencing sequence targeted to VSV-G.
44. A method of transducing a population of peripheral blood lymphocytes with a Lentiviral vector, where the lymphocyte population is not purified into subpopulations prior to transduction with the Lentiviral vector.
45. A method to treat cancer using Lentiviral vectors, where stem cells are treated with a Lentiviral vector expressing a cytotoxic element that is operably linked to an endothelial specific promoter, and where the stem cells are infused into a cancer patient.

## Claims

1. An in vitro method for manufacturing a polypeptide of interest that is secreted from a host cell line, comprising transducing a host cell line with a lentivirus transduction vector in sufficient numbers to form a transduced host cell line comprising 10-100 vectors per cell, wherein said vector comprises an expressible heterologous polynucleotide coding for a secreted heterologous polypeptide of interest, and culturing said transduced host cell line under conditions effective to produce and secrete said polypeptide of interest.

2. The method of claim 1, wherein said host cell line is transduced with a lentivirus transduction vector that comprises two or more different expressible heterologous polynucleotides coding for two or more different polypeptides, or is transduced with a plurality of lentivirus transduction vectors, wherein each vector comprises a different heterologous polynucleotide coding for a different polypeptide, optionally wherein each of said heterologous polynucleotides codes for at least one polypeptide of a virus, which when expressed in said host cell line, self-assemble into a viral-like particle (VLP).

3. The method of claim 2, wherein each of said heterologous polynucleotides codes for at least one polypeptide of a virus, which when expressed in said host cell line, self-assemble into a VLP.

4. The method of claims 2 or 3, wherein at least one of said heterologous polynucleotides codes for a viral envelope protein.

5. The method of any one of claims 1-4, wherein at least one of said heterologous polynucleotides codes for a hemagglutinin or a neuraminadase polypeptide of an influenza virus.

6. The method of any one of claims 1-4, wherein said host cell line is transduced with one or more lentivirus transduction vectors comprising polynucleotides coding for hemagglutinin, neuraminadase, and matrix polypeptides of an influenza virus.

7. The method of any one claims 1-6, wherein said host cell line is a CHO or a 293 cell line.

8. The method of any one of claims 1-7, wherein said lentivirus transduction vector further comprises a second gene to increase the production of the polypeptide of interest.

9. A polypeptide produced by the method of any one of claims 1-8.

10. The polypeptide of claim 9, wherein said polypeptide is an antigen useful as a vaccine.

11. A VLP produced by the method of any one of claims 2-8.

12. A VLP comprising an influenza hemagglutinin protein and an influenza neuraminidase protein.

13. The VLP of claim 12 further comprising an influenza matrix protein.

14. A vaccine comprising the polypeptide of claim 9 or the VLP of any one of claims 11-13.
